# EUROPEAN PATENT APPLICATION

(11) **EP 4 317 407 A1**
(43) Date of publication of application: **07.02.2024**
(21) Application number: 22795647.1
(22) Date of filing: 20.04.2022
(51) Int. Cl.: C12M 3/00, C12N 5/071, C12N 5/0735

(54) **CELL CULTURE SUBSTRATE AND METHOD FOR PRODUCING SAME, METHOD FOR INDUCING DIFFERENTIATION OF PLURIPOTENT STEM CELL, AND CELL CULTURE KIT**

(30) Priority: 27.04.2021 JP 2021075266
(71) Applicant: Tosoh Corporation, Yamaguchi 746-8501 (JP)
(72) Inventor: IMAIZUMI Yu, Yokkaichi-shi, Mie 510-8540 (JP); KADOTA Junpei, Yokkaichi-shi, Mie 510-8540 (JP); KUNO Goshi, Yokkaichi-shi, Mie 510-8540 (JP)
(74) Representative: Novagraaf International SA
(86) International application number: PCT/JP2022/018285
(87) International publication number: WO 2022/230734

(57) **Abstract**

A cell culture substrate including: a substrate; and a layer containing hydrophilic polymer with a layer thickness of 5 to 2,000 nm covering at least a part of a surface of the substrate, in which the hydrophilic polymer contain a phosphorylcholine group or a hydroxyl group, the cell culture substrate has regions (A) below and regions (B) below, and an unevenness height at a boundary between each of the regions (A) and each of the regions (B) is 1 to 500 nm.
(A): Island-shaped region of 0.001 to 5 mm² in area with cell adhesiveness and cell proliferation properties
(B): Region which is adjacent to region (A) and has no cell adhesiveness or cell proliferation properties

## Description

### Technical Field

The present invention relates to a cell culture substrate and a method for producing the same, a method for inducing differentiation of pluripotent stem cells, and a cell culture kit.

### Background Art

Pluripotent stem cells such as embryonic stem cells (ES cells) and induced pluripotent stem cells (iPS cells) are cells that have an ability to differentiate into various tissues of a living body (pluripotency) and have been attracted significant attention as a cell source for the regenerative medical field and drug discovery screening. To apply pluripotent stem cells to regenerative medicine and drug discovery screening, it is necessary to differentiate pluripotent stem cells into target cells, but at this time, this requires formation of cell aggregates of the pluripotent stem cells. In addition, although pluripotent stem cells can differentiate into various cells, it is known that the optimal size of the cell aggregates differs depending on the types of cells after differentiation, and it is desirable to control the sizes thereof and produce cell aggregates of uniform size.

As a method for forming cell aggregates, a method for allowing pluripotent stem cells to spontaneously form aggregates using a substrate to which pluripotent stem cells do not adhere is known in the related art (for example, refer to Patent Literature 1). Although this method is excellent in mass productivity of cell aggregates, there is a problem that cell aggregates of uniform size cannot be obtained.

As a method for forming cell aggregates of uniform size, a method of using a cell culture substrate having fine unevenness on its surface is known (for example, refer to Patent Literature 2).

### Citation List

### Patent Literature

[Patent Literature 1] Japanese Unexamined Patent Publication No. H8-140673
[Patent Literature 2] Japanese Unexamined Patent Publication No. 2015-073520

### Summary of Invention

### Technical Problem

A cell culture substrate provided with fine unevenness as disclosed in Patent Literature 2 has a problem in that it has poor mass productivity and it is unsuitable for use in forming a large amount of cell aggregates. In addition, since cells are forcibly aggregated within the fine unevenness, there is a problem that dead cells are likely to be mixed into the cell aggregates. In addition, the present inventors have found that, when a medium is brought into contact with the cell culture substrate having fine unevenness, air bubbles are likely to be incorporated into the fine unevenness on the surface, and there is a problem that it is necessary to remove the air bubbles before starting culture. To remove the air bubbles, it is usually necessary to repeatedly aspirate and discharge the medium using a pipetter, resulting in poor workability.

An object of the present invention is to provide a cell culture substrate which can form cell aggregates, can increase viability of cells in the cell aggregates, and eliminates the need for removing air bubbles during culture, and a method for producing the same. Another object of the present invention is to provide a method for inducing differentiation of pluripotent stem cells which has an excellent efficiency in inducing differentiation into three germ layer cells and in which the cell culture substrate is used.

### Solution to Problem

The present invention relates to a cell culture substrate including: a substrate; and a layer containing a hydrophilic polymer with a layer thickness of 5 to 2,000 nm covering at least a part of a surface of the substrate, in which the above-described hydrophilic polymer contains a phosphorylcholine group or a hydroxyl group, the cell culture substrate has regions (A) below and regions (B) below, and an unevenness height at a boundary between each of the regions (A) and each of the regions (B) is 1 to 500 nm.
(A): Island-shaped region of 0.001 to 5 mm² in area with cell adhesiveness and cell proliferation properties
(B): Region which is adjacent to region (A) and has no cell adhesiveness or cell proliferation properties

The present invention also relates to a method for producing the above-described cell culture substrate, the method including: Step (1) of coating at least a part of the surface of the substrate with a composition containing a UV-reactive hydrophilic polymer to form a layer containing the hydrophilic polymer; Step (2) of irradiating the layer containing the hydrophilic polymer with UV light to immobilize the layer containing the hydrophilic polymer on the surface of the substrate; and Step (3) of subjecting a part of the surface of the immobilized layer containing the hydrophilic polymer to a plasma treatment to form the regions (A) in the plasma-treated portions.

The present invention also relates to a method for producing the above-described cell culture substrate, the method including: Step (1') of using a substrate made of a polymer containing an aromatic hydrocarbon group in a repeating unit and subjecting the surface of the above-described substrate to a plasma treatment to form the above-described regions (A) in the plasma-treated portions; Step (2') of coating at least a part of the surface of the above-described substrate with a composition containing a UV-reactive hydrophilic polymer to form the layer containing the above-described hydrophilic polymer; Step (3') of irradiating a part of the layer containing the above-described hydrophilic polymer with UV light to immobilize the part of the layer containing the above-described hydrophilic polymer on the surface of the above-described substrate; and Step (4') of washing the above-described hydrophilic polymer with a solvent to dissolve hydrophilic polymer that have not been immobilized on the surface and remove them from the surface of the above-described substrate.

The present invention further relates to a method for inducing differentiation of pluripotent stem cells, the method including: Step (i) of seeding the above-described cell culture substrate with pluripotent stem cells; Step (ii) of culturing the pluripotent stem cells to form hemispherical cell aggregates with a height-to-diameter ratio of 0.2 to 0.8; and Step (iii) of inducing differentiation of the cell aggregates to form cell aggregates of three germ layer cells.

In addition, the present invention relates to a cell culture kit including: the above-described cell culture substrate; and a block copolymer containing a water-insoluble block segment and a temperature-responsive block segment or a coating agent containing the above-described block copolymer.

### Advantageous Effects of Invention

According to the present invention, it is possible to provide a cell culture substrate which can form cell aggregates, can increase viability of cells in the cell aggregates, and eliminates the need for removing air bubbles during culture, and a method for producing the same. According to the present invention, it is also possible to provide a method for inducing differentiation of pluripotent stem cells using the cell culture substrate.

### Brief Description of Drawings

FIG. 1 is a schematic diagram (cross-sectional view) of a cell culture substrate according to one embodiment.
FIG. 2 is a schematic diagram (perspective view) of a substrate having a layer containing a hydrophilic polymer formed on its surface after Step (1) in a production method according to one embodiment.
FIG. 3 is a schematic diagram (perspective view) of the cell culture substrate after Step (3) in a production method according to one embodiment.
FIG. 4 is a schematic diagram (perspective view) of the cell culture substrate after Step (4) in a production method according to one embodiment.
FIG. 5 shows phase-contrast microscopic images of cells on "day 0 of differentiation," "day 43 of differentiation," and "day 64 of differentiation" in a test for inducing differentiation of human iPS cells into intestinal epithelium cells (Example 8).

### Description of Embodiments

Hereinafter, embodiments of the present invention will be described in detail. The present invention is not limited to the following embodiments, and can be implemented by modifying the following embodiments within the range in which the above-described effects can be obtained.

The term "cell aggregate" in the present specification means a three-dimensional aggregate of cells formed by aggregation of a plurality of cells. The three-dimensional aggregate may have an ellipsoidal shape such as a spherical shape or may be a shape such as a hemispherical shape. These shapes may be shapes with gaps formed by folding sheet-like cells, or may be hollow shapes.

The term "temperature-responsive" in the present specification indicates that the degree of hydrophilicity/hydrophobicity changes with change in temperature. Furthermore, the boundary temperature at which the degree of hydrophilicity/hydrophobicity changes is referred to as "response temperature."

The term "living body-derived substance" in the present specification means a substance existing in a body of an organism, or a chemically synthesized substance which is equivalent to the substance. Substances existing in a body of an organism may be natural substances or may be artificially synthesized through gene recombination techniques and the like. Although there is no particular limitation on the living body-derived substances, examples thereof include: nucleic acids, proteins, and polysaccharides which are basic materials constituting a living body; nucleotides, nucleosides, amino acids, and various saccharides which are constituent elements thereof; lipids; vitamins; and hormones.

The term "cell adhesiveness" in the present specification indicates ease of adhesion to a cell culture substrate or a substrate at a culture temperature, and the expression "having cell adhesiveness" indicates that cells can adhere to a cell culture substrate or a substrate at a culture temperature directly or via a living body-derived substance. In addition, the expression "having no cell adhesiveness" indicates that calls cannot adhere to a cell culture substrate or a substrate at a culture temperature.

The term "cell proliferation properties" in the present specification indicates ease of proliferation of cells at a culture temperature, and the expression "having cell proliferation properties" indicates that cells can proliferate at a culture temperature. In addition, the expression "having no cell proliferation properties" indicates that cells cannot proliferate at a culture temperature. The expression "high cell proliferation properties" indicates that cells proliferate into a larger number of cells when compared during the same culture period.

The "three germ layer cells" in the present specification means at least one selected from the group consisting of endoderm cells, ectoderm cells, and mesoderm cells.

A cell culture substrate according to the present embodiment includes: a substrate; and a layer containing a hydrophilic polymer with a layer thickness of 5 to 2,000 nm covering at least a part of a surface of the substrate. Here, the hydrophilic polymer contains a phosphorylcholine group or a hydroxyl group. In addition, the cell culture substrate according to the present embodiment has regions (A) below and regions (B) below, and an unevenness height at a boundary between each of the regions (A) and each of the regions (B) is 1 to 500 nm.
(A): Island-shaped region of 0.001 to 5 mm² in area with cell adhesiveness and cell proliferation properties
(B): Region which is adjacent to region (A) and has no cell adhesion or cell proliferation properties

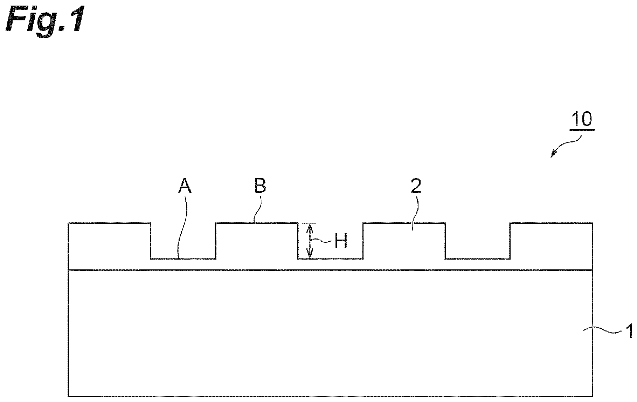

FIG. 1 is a schematic diagram (cross-sectional view) of a cell culture substrate according to one embodiment. A cell culture substrate 10 shown in FIG. 1 includes: a substrate 1; and a layer 2 containing a hydrophilic polymer covering a surface of the substrate 1 (the layer thickness is, for example, 5 to 2,000 nm). In FIG. 1, A and B respectively indicate a region (A) and a region (B). In addition, in FIG. 1, H indicates an unevenness height at a boundary between the region (A) and the region (B). The layer thickness of the layer 2 containing a hydrophilic polymer means a distance between the surface of the region (B) (In FIG. 1, the region indicated by B) and the surface where the layer 2 containing a hydrophilic polymer comes into contact with the substrate 1. In addition, in the cell culture substrate 10 shown in FIG. 1, the surface of the region (A) is a layer containing a hydrophilic polymer, but is not limited thereto. For example, the surface of the region (A) may be the substrate 1.

The substrate used in the cell culture substrate according to the present embodiment is not particularly limited, but is preferably made of at least one selected from the group consisting of polystyrene, polyethylene, polyethylene terephthalate, polycarbonate, cycloolefin polymer, cellulose acetate, nitrocellulose, and polyvinylidene fluoride, more preferably made of at least one selected from the group consisting of polystyrene, polyethylene terephthalate, polycarbonate, and cycloolefin polymer, still more preferably made of at least one selected from polystyrene, polyethylene terephthalate, and polycarbonate, and most preferably formed of polystyrene or polycarbonate. Examples of commercially available products of cycloolefin polymer include ZEONEX (manufactured by Zeon Corporation), ZEONOR (manufactured by Zeon Corporation), and ARTON (manufactured by JSR Corporation).

Since it is suitable for observing cells cultured on the cell culture substrate with a high-magnification phase-contrast microscope, the refractive index of the substrate measured using a D-line (wavelength of 589 nm) is preferably 1.4 to 1.6, more preferably 1.45 to 1.6, and particularly preferably 1.5 to 1.55. When the refractive index of the substrate is within these ranges, the spherical aberration in the phase-contrast microscopic observation of the cells can be reduced, and a clear phase-contrast image can be obtained. In addition, to reduce the spherical aberration, the thickness of the substrate is preferably 0.5 mm or less, more preferably 0.4 mm or less, particularly preferably 0.3 mm or less, and most preferably 0.2 mm or less. On the other hand, since it is suitable for suppressing bending of the substrate during the microscopic observation, which causes the entire observation range to be out of focus, the thickness of the substrate is preferably 0.01 mm or more, more preferably 0.05 mm or more, particularly preferably 0.1 mm or more, and most preferably 0.15 mm or more.

The refractive index and thickness of the substrate measured using the D-line (wavelength of 589 nm) are preferably respectively 1.4 to 1.6 and 0.01 mm to 0.5 mm, more preferably respectively 1.45 to 1.6 and 0.05 mm to 0.4 mm, still more preferably respectively 1.45 to 1.55 and 0.1 mm to 0.3 mm, and particularly preferably respectively 1.5 to 1.55 and 0.15 mm to 0.2 mm. When the refractive index and thickness of the substrate is within these ranges, the phase-contrast image of cells becomes clearer.

The fluorescence intensities (autofluorescence intensities) of the substrate at excitation wavelengths of 350 nm, 488 nm, and 647 nm (when irradiated with excitation light beams having these wavelengths) are preferably smaller than fluorescence intensities (autofluorescence intensities) of a 1.2 mm-thick polystyrene plate excited at light beams having the same excitation wavelengths, more preferably 80% or less of the fluorescence intensities of the 1.2 mm-thick polystyrene plate, particularly preferably 50% or less of the fluorescence intensities of the 1.2 mm-thick polystyrene plate, and most preferably 10% or less of the fluorescence intensities of the 1.2 mm-thick polystyrene plate because they are suitable for observing cells cultured on the cell culture substrate with a high-magnification fluorescence microscope. Fluorescent dyes excited at excitation wavelengths of 350 nm, 488 nm, and 647 nm are frequently used in fluorescence observation of cells. When the autofluorescence intensities of the substrate at these wavelengths are a certain value or less, a clear fluorescence image of cells can be obtained.

The shape of the substrate is not particularly limited, and the substrate may have a planar shape like a plate or a film, or may have a shape of a fiber, porous particles, a porous membrane, a hollow fiber, or the like. In addition, the substrate may have a shape of a container (such as a cell culture plate such as a Petri dish, a flask, a plate, and a bag) used generally for cell culture and the like. From the viewpoint of easiness of culture operation, the substrate preferably has a planar shape like a plate or a film or a planar porous membrane shape.

The layer thickness of the layer containing a hydrophilic polymer is 5 to 2,000 nm. Since the layer thickness of the layer of the hydrophilic polymer is 5 to 2,000 nm, cells can adhere only to and proliferate only in the region (A), and cells are likely to be collected in the region (A) due to cell migration, thereby increasing the viability of cells in cell aggregates. In a case where the layer thickness is less than 5 nm, cells can also proliferate in the region (B), making it impossible to form cell aggregates. In a case where the layer thickness exceeds 2,000 nm, cells cannot migrate, thereby reducing the viability of cells contained in cell aggregates. Here, the term "layer thickness" of the layer of the hydrophilic polymer indicates a length in the out-of-plane direction from an interface between the substrate and the layer of the hydrophilic polymer to an interface (excluding the region (A)) of the layer of the hydrophilic polymer on the opposite side of the substrate. In a range where the layer thickness exceeds 10 nm, a cross-sectional image can be measured with a transmission electron microscope using an ultra-thin slice of a cell culture substrate produced using a microtome, and a distance can be calculated by measuring a distance at 10 randomly selected points, and averaging them. In addition, in the range of the layer thickness of 10 nm or less, the measurement can be performed using an ellipsometer. The layer thickness is more preferably 10 nm or more, still more preferably 50 nm or more, and most preferably 100 nm or more because this is suitable for suppressing cell adhesion to the region (B). In addition, the layer thickness is more preferably 1,000 nm or less, still more preferably 500 nm or less, and most preferably 200 nm or less because they are suitable for increasing the viability of cells in cell aggregates by collecting the cells in the region (A) due to cell migration.

The hydrophilic polymer contains a phosphorylcholine group or a hydroxyl group. When the hydrophilic polymer contains a phosphorylcholine group or a hydroxyl group, it is possible to make the region coated with the hydrophilic polymer a region to which cells do not adhere. In addition, since such hydrophilic polymer does not need to be completely decomposed and removed, only a short period of time of a weak plasma treatment can make the region cell-adhesive and cell-proliferative, and degraded hydrophilic polymer is less likely to be mixed into the cells. The types of hydrophilic polymer are not particularly limited except that these contain a phosphorylcholine group or a hydroxyl group, and examples of commercially available products thereof include Lipidure (R) CM5206 (manufactured by NOF Corporation), Lipidure (R) CM2001 (manufactured by NOF Corporation), and BIOSURFINE (R)-AWP (manufactured by Toyo Gosei Co., Ltd.). In addition, as commercially available substrates coated with hydrophilic polymer, PrimeSurface (R) (manufactured by Sumitomo Bakelite Co., Ltd.), Ez-BindShut (R) (manufactured by AGC Techno Glass Co., Ltd.), and EZ-BindShut II (R) (manufactured by AGAGC Techno Glass Co., Ltd.) can be suitably used.

A hydrophilic polymer preferably contains a compound represented by General Formula (1) below, a compound represented by General Formula (2), or a compound represented by General Formula (3) below. 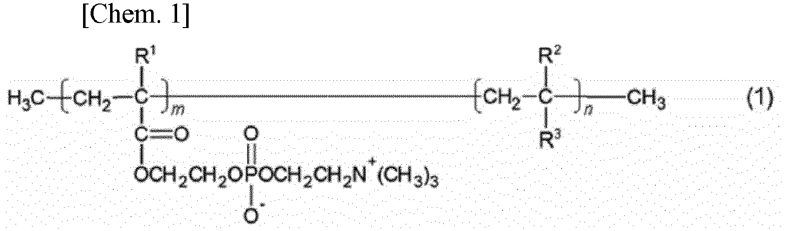
[In General Formula (1), R¹ and R² each independently represent a hydrogen atom or a methyl group, R³ represents a hydrogen atom or an arbitrary organic group, and m and n each independently represent a positive integer.] 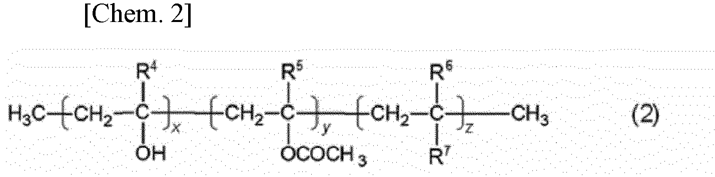
[In General Formula (2), R⁴, R⁵, and R⁶ each independently represent a hydrogen atom or a methyl group, R⁷ represents a hydrogen atom or an arbitrary organic group, and x, y, and z each independently represent a positive integer.] 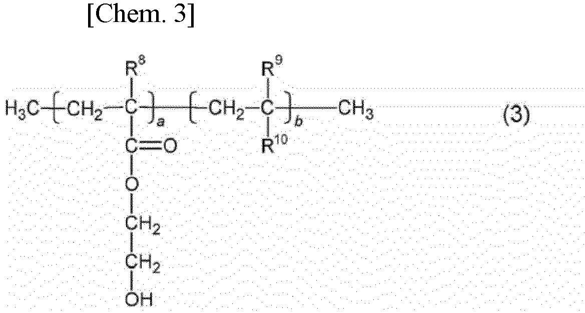
[In General Formula (3), R⁸ and R⁹ each independently represent a hydrogen atom or a methyl group, R¹⁰ represents a hydrogen atom or an arbitrary organic group, and a and b each independently represent a positive integer.]

When the hydrophilic polymer contains the compound represented by General Formula (1) above, the compound represented by General Formula (2) above, or the compound represented by General Formula (3) above, adhesion and proliferation of cells are likely to occur, and they are suitable for forming uniformly shaped cell aggregates in the region (A). In General Formula (1), General Formula (2), and General Formula (3) above, since R³R⁷ and R¹⁰ are suitable for immobilizing the hydrophilic polymer on a substrate, these are preferably hydrophobic groups or UV-reactive functional groups. As hydrophobic groups, for example, linear or cyclic alkyl groups such as a methyl group, an ethyl group, a propyl group, a butyl group, and a cyclohexyl group can be suitably used. In addition, examples of UV-reactive functional groups include an azide group, an acrylate group, a methacrylate group, and an epoxy group, and an azide group can be suitably used.

The region (A) is an island-shaped region of 0.001 to 5 mm² in area with cell adhesiveness and cell proliferation properties. Due the island-shaped region of 0.001 to 5 mm² in area, cell aggregates having a uniform particle diameter can be formed when cells are cultured. In addition, the area is preferably 0.005 to 1 mm², more preferably 0.01 to 0.5 mm², still more preferably 0.015 to 0.25 mm², and most preferably 0.02 to 0.2 mm² because this is suitable for forming cell aggregates suitable for applications such as induction of differentiation of pluripotent stem cells.

The fact that the region (A) is an island-shaped region indicates that the region (A) exists independently of regions other than the region (A). Since the region (A) has an island shape with cell adhesiveness and cell proliferation properties, viable cells can be concentrated in the region (A), thereby producing cell aggregates. In a case where the region (A) does not have the island shape, if it has, for example, a stripe structure or the like, cell aggregates cannot be produced. The island shape is not particularly limited and can be appropriately set according to the shapes of target cell aggregates, and examples thereof include a circle, an ellipse, a polygon, and closed shapes formed by a straight line and curved line. In addition, the shape of the island shape is preferably a circle, an ellipse, or a polygon, more preferably a circle, an ellipse, or a rectangle, still more preferably a circle, an ellipse, or a square, and most preferably a circle or an ellipse because this is suitable for producing cell aggregates having a shape close to a sphere.

The standard deviation/average area of the area of the region (A) is preferably 80% or less, more preferably 50% or less, still more preferably 20% or less, and most preferably 5% or less because this is suitable for producing uniform-sized and uniform-shaped cell aggregates.

The aspect ratio of the island shape is preferably 5 or less, more preferably 2 or less, still more preferably 1.5 or less, and most preferably 1.1 or less because this is suitable for producing cell aggregates having a shape close to a sphere. Here, the "aspect ratio" indicates a major axis-to-minor axis ratio which is a ratio of the maximum diameter (major axis) to the minimum diameter (minor axis) of the shape.

In addition, since it is suitable for forming cell aggregates suitable for culturing pluripotent stem cells and inducing differentiation of the pluripotent stem cells into endoderm cells or ectoderm cells, it is preferable that the area of the region (A) be 0.005 to 0.2 mm² and the number of regions (A) be 200 to 1,000 regions/cm² based on the total area of the regions (A) and regions (B), it is more preferable that the area of the region (A) be 0.01 to 0.15 mm² and the number of regions (A) be 250 to 800 regions/cm² based on the total area of the regions (A) and regions (B), it is still more preferable that the area of the region (A) be 0.02 to 0.1 mm² and the number of regions (A) be 300 to 600 regions/cm² based on the total area of the regions (A) and regions (B), and it is most preferable that the area of the region (A) be 0.03 to 0.05 mm² and the number of regions (A) be 300 to 400 regions/cm² based on the total area of the regions (A) and regions (B).

Since it is suitable for forming cell aggregates suitable for culturing pluripotent stem cells and inducing differentiation of the pluripotent stem cells into mesoderm cells, it is preferable that the area of the region (A) be 0.2 to 2 mm² and the number of regions (A) be 3 to 15 regions/cm² based on the total area of the regions (A) and regions (B), it is more preferable that the area of the region (A) be 0.3 to 1.5 mm² and the number of regions (A) be 4 to 12 regions/cm² based on the total area of the regions (A) and regions (B), it is still more preferable that the area of the region (A) be 0.4 to 1 mm² and the number of regions (A) be 5 to 10 regions/cm² based on the total area of the regions (A) and regions (B), and it is most preferable that the area of the region (A) be 0.5 to 0.7 mm² and the number of regions (A) be 6 to 8 regions/cm² based on the total area of the regions (A) and regions (B).

In addition, the minimum distance between the regions (A) is preferably 500 to 10,000 µm, more preferably 1,000 to 8,000 µm, still more preferably 2,000 to 5,000 µm, and most preferably 3,000 to 4,000 µm because this is suitable for increasing the oxygen concentration around cells and increasing the viability of cell aggregates.

The regions (A) can be formed, for example, by forming a layer containing a ahydrophilic polymer on the surface of a substrate, followed by modifying a part of the surface of the layer containing a hydrophilic polymer through a corona treatment, a UV treatment, or a plasma treatment. The modification is preferably performed through a plasma treatment. Since the regions (A) are regions in which the surface of the layer containing a hydrophilic polymer is modified through a plasma treatment or the like, the regions (A) have cell adhesiveness and cell proliferation properties. Since the regions (A) can be patterned through a plasma treatment or the like in a short period of time, the mass productivity of a cell culture substrate can be increased.

In addition, the ratio of a peak intensity at 287 eV to a peak intensity at 285 eV in a C1s spectrum when XPS (X-ray photoelectron spectroscopy) was measured for the regions (A) is preferably greater than the ratio of a peak intensity at 287 eV to a peak intensity at 285 eV in a C1s spectrum of XPS measurement for the regions (B) by 0.05 or more. In this case, since the difference in cell proliferation properties between the regions (A) and the regions (B) can be increased, a large number of cells can proliferate in the regions (A) and uniform cell aggregates are likely to be formed in the regions (A). The ratio of a peak intensity at 287 eV to a peak intensity at 285 eV in a C1s spectrum of XPS measurement for the regions (A) is more preferably greater than the ratio of a peak intensity at 287 eV to a peak intensity at 285 eV in a C1s spectrum of XPS measurement for the regions (B) by 0.07 or more, still more preferably by 0.1 or more, and most preferably by 0.15 or more because this is suitable for forming uniform cell aggregates.

A method for adjusting the ratio of a peak intensity at 287 eV to a peak intensity at 285 eV in a C1s spectrum of XPS measurement to within the above-described ranges is not particularly limited, but a method for performing a plasma treatment, a corona treatment, a UV treatment, or the like only on a part (a portion that is to become a region (A)) of the layer containing a hydrophilic polymer formed on the surface of a substrate is preferable, a method for performing a plasma treatment or a corona treatment is still more preferable, and a method for performing a plasma treatment is particularly preferable. Examples of methods for performing a plasma treatment or the like only on the portion that is to become a region (A) include a method for covering the layer containing a hydrophilic polymer formed on the surface of the substrate with a mask produced through laser processing to perform a plasma treatment or the like from above the mask. The conditions such as plasma intensity and plasma irradiation time can be appropriately adjusted so that the difference between the ratio of a peak intensity at 287 eV to a peak intensity at 285 eV in a C1s spectrum of XPS measurement for the regions (A) and the ratio of a peak intensity at 287 eV to a peak intensity at 285 eV in a C1s spectrum of XPS measurement for the regions (B) falls within the above-described ranges. In the case of the plasma treatment, use of a gas containing oxygen and nitrogen as an introduction gas makes it easier for the difference therebetween to fall within the above-described ranges.

Since the regions (A) are suitable for peeling off cultured cell aggregates, these may have temperature responsiveness. In a case where the regions (A) have temperature responsiveness, cells can be cultured at a temperature close to the body temperature when the cells are cultured on a cell culture substrate. Therefore, the response temperature is preferably 50°C or lower and more preferably 35°C or lower. In addition, , the response temperature is particularly preferably 25°C or lower because this is suitable for suppressing detachment of cells when performing operations such as medium exchange during culture. Furthermore, since it is possible to form cell aggregates through a cooling operation at a temperature at which cells are not damaged, the response temperature is preferably 4°C or higher, more preferably 10°C or higher, and still more preferably 15°C or higher.

In a case where the regions (A) have temperature responsiveness, for example, a layer containing a temperature-responsive polymer with a layer thickness of 1 to 100 nm may be further provided on the surface of the layer containing a hydrophilic polymer (surface containing the regions (A) and the regions (B)). By providing the layer containing a temperature-responsive polymer with a layer thickness of 1 to 100 nm, it is possible to impart temperature responsiveness to the regions (A) without impairing the properties of the regions (A) and the regions (B) formed on the surface of the layer containing a hydrophilic polymer. The layer thickness of the layer containing a temperature-responsive polymer is more preferably 3 to 50 nm, still more preferably 5 to 40 nm, and most preferably 10 to 35 nm because this is suitable for imparting temperature responsiveness to the regions (A) without impairing the properties of the regions (A) and the regions (B). The suitable thickness of the temperature-responsive polymer layer from which cells can be further peeled off due to temperature responsiveness after culture and collected without impairing the cell proliferation properties can also vary depending on cells to be cultured and can be appropriately adjusted to the above-exemplified ranges of the layer thickness.

The temperature-responsive polymer is preferably block copolymer containing a water-insoluble block segment and a temperature-responsive block segment. Since the temperature-responsive polymer is such block copolymer, the mass productivity of a cell culture substrate can be increased and the temperature-responsive polymer can be suppressed from being mixed into produced cell aggregates. The structural unit proportion of temperature-responsive block segment contained in the temperature-responsive polymer is preferably 70 wt% or more, more preferably 80 wt% or more, particularly preferably 90 wt% or more, and most preferably 92 wt% or more because this is suitable for promptly peeling off cell aggregates from the cell culture substrate.

In addition, since the temperature-responsive polymer is block copolymer having a water-insoluble block segment and a temperature-responsive block segment, temperature responsiveness can be imparted to the surface of the cell culture substrate through a simple technique of adding dropwise a solution containing a temperature-responsive polymer to the surface of the cell culture substrate and drying the solution. In addition, since the temperature-responsive polymer layer formed at this time has a preferred thickness, even if the entire surface of the layer containing a hydrophilic polymer is coated with the temperature-responsive polymer, the properties of the above-described regions (A) and regions (B) are less likely to be impaired. In a case where a cell culture kit containing the cell culture substrate of the present invention and a temperature-responsive polymer which are block copolymer or a coating agent containing the above-described block copolymer is used, the thickness of the temperature-responsive polymer layer can be simply adjusted by a researcher performing culture depending on the types of cells.

The coating agent may contain a solvent. Examples of solvents that can be contained in the coating agent include water, an organic solvent, or a mixture thereof. Examples of organic solvents include: alcohols such as methanol, ethanol, 1-propanol, 2-propanol, and 1-butanol; acetonitrile; formamide; N,N-dimethylformamide; dimethyl sulfoxide; tetrahydrofuran; 1,4-dioxane; and methyl ethyl ketone. Since it is suitable for making the film thickness of a coating uniform, it is preferable to use a mixed solvent of water and alcohols. The content of block copolymer having a water-insoluble block segment and a temperature-responsive block segment can be set to 0.1 to 50 weight%, 0.2 to 10 weight%, or 0.5 to 5 weight% based on the total mass of the coating agent.

The coating agent may contain other components than the solvent and the block copolymer having a water-insoluble block segment and a temperature-responsive block segment. Examples of other components include components, such as polymer consisting of only water-insoluble block segment, for enhancing cell adhesiveness.

Examples of monomer units constituting a temperature-responsive block segment include: (meth)acrylamide compounds such as acrylamide and methacrylamide; N-alkyl-substituted (meth)acrylamide derivatives such as N,N-diethyl acrylamide, N-ethyl acrylamide, N-n-propyl acrylamide, N-n-propyl methacrylamide, N-isopropyl acrylamide, N-isopropyl methacrylamide, N-cyclopropyl acrylamide, N-cyclopropyl methacrylamide, N-t-butyl acrylamide, N-ethoxyethyl acrylamide, N-ethoxyethyl methacrylamide, N-tetrahydrofurfuryl acrylamide, and N-tetrahydrofurfuryl methacrylamide; N,N-dialkyl-substituted (meth)acrylamide derivatives such as N,N-dimethyl (meth)acrylamide, N,N-ethylmethyl acrylamide, and N,N-diethylacrylamide; (meth)acrylamide derivatives, such as 1-(1-oxo-2-propenyl)-pyrrolidine, 1-(1-oxo-2-propenyl)-piperidine, 4-(1-oxo-2-propenyl)-morpholine, 1-(1-oxo-2-methyl-2-propenyl)-pyrrolidine, 1-(1-oxo-2-methyl-2-propenyl)-piperidine, and 4-(1-oxo-2-methyl-2-propenyl)-morpholine, having a cyclic group; vinyl ethers such as methyl vinyl ether; and proline derivatives such as N-proline methyl ester acrylamide. Since it is suitable for setting the response temperature to 0°C to 50°C, N,N-diethyl acrylamide, N-n-propyl acrylamide, N-isopropyl acrylamide, N-n-propyl methacrylamide, N-ethoxyethyl acrylamide, N-tetrahydrofurfuryl acrylamide, and N-tetrahydrofurfuryl methacrylamide are preferable, N-n-propyl acrylamide and N-isopropyl acrylamide are more preferable, and N-isopropyl acrylamide is particularly preferable. In addition, N-n-propyl acrylamide and N-proline methyl ester acrylamide are preferable because they are suitable for setting the response temperature of the block copolymer to a temperature lower than room temperature in a case of using a medium at room temperature during medium exchange in a culture operation.

Examples of monomer units constituting a water-insoluble block segment include n-butyl acrylate, n-butyl methacrylate, isobutyl acrylate, isobutyl methacrylate, t-butyl acrylate, t-butyl methacrylate, n-hexyl acrylate, n-hexyl methacrylate, n-octyl acrylate, n-octyl methacrylate, n-decyl acrylate, n-decyl methacrylate, n-dodecyl acrylate, n-dodecyl methacrylate, n-tetradecyl acrylate, and n-tetradecyl methacrylate. In addition, reactive groups are preferable because they are suitable for firmly immobilizing the block copolymer on the substrate, and examples thereof include 4-azidophenyl acrylate, 4-azidophenyl methacrylate, 2-((4-azidobenzoyl)oxy) ethyl acrylate, and 2-((4-azidobenzoyl)oxy) ethyl methacrylate. Furthermore, a structure having an aromatic ring is preferable because this is suitable for increasing cell proliferation properties, and examples thereof include 2-hydroxyphenyl acrylate, 2-hydroxyphenyl methacrylate, 3-hydroxyphenyl acrylate, 3-hydroxyphenyl methacrylate, 4-hydroxyphenyl acrylate, 4-hydroxyphenyl methacrylate, N-(2-hydroxyphenyl) acrylamide, N-(2-hydroxyphenyl) methacrylamide, N-(3-hydroxyphenyl) acrylamide, N-(3-hydroxyphenyl) methacrylamide, N-(4-hydroxyphenyl) acrylamide, N-(4-hydroxyphenyl) methacrylamide, and styrene.

Water-insoluble block segment may also contain repeating units that control the response temperature of the block copolymer. As repeating units controlling the response temperature of the block copolymer, hydrophilic or hydrophobic components can be exemplified, and are not particularly limited. Examples thereof include: those having amino groups such as 2-dimethylaminoethyl acrylate, 2-dimethylaminoethyl methacrylate, 2-diethylaminoethyl acrylate, 2-diethylaminoethyl methacrylate, N-[3-(dimethylamino)propyl] acrylamide; those having betaine such as N-(3 - sulfopropyl)-N-methacryloyloxy ethyl-N,N-dimethylammonium betaine and N-methacryloyloxyethyl-N,N-dimethylammonium-α-N-methyl carboxybetaine; those having methoxyethyl groups and polyethylene glycol groups such as hydroxyethyl acrylate, hydroxyethyl methacrylate, N-(2-hydroxyethyl) acrylamide, polyethylene glycol monoacrylate, polyethylene glycol monomethacrylate, polypropylene glycol monoacrylate, polypropylene glycol monomethacrylate, methoxypolyethylene glycol monoacrylate, methoxypolyethylene glycol monomethacrylate, diethylene glycol monomethyl ether acrylate, diethylene glycol monomethyl ether methacrylate, diethylene glycol monoethyl ether acrylate, diethylene glycol monoethyl ether methacrylate, 2-methoxyethyl acrylate, 2-methoxyethyl methacrylate, 2-ethoxyethyl acrylate, 2-ethoxyethyl methacrylate, 3-butoxyethyl acrylate, 3-butoxyethyl methacrylate, 3-butoxyethyl acrylamide, furfuryl acrylate, furfuryl methacrylate, tetrahydrofurfuryl acrylate, and tetrahydrofurfuryl methacrylate; those having acrylate groups such as methoxymethyl acrylate, methoxymethyl methacrylate, 2-ethoxymethyl acrylate, 2-ethoxymethyl methacrylate, 3-butoxymethyl acrylate, 3-butoxymethyl methacrylate, and 3-butoxymethyl acrylamide; and those having phosphorylcholine groups such as 2-methacryloyloxyethyl phosphorylcholine, 2-acryloyloxyethyl phosphorylcholine, 3-(meth)acryloyloxypropyl phosphorylcholine, 4-(meth)acryloyloxybutyl phosphorylcholine, 6-(meth)acryloyloxyhexyl phosphorylcholine, 10-(meth)acryloyloxy decyl phosphorylcholine, ω-(meth)acryloyl (poly)oxyethylene phosphorylcholine, 2-acrylamidoethyl phosphorylcholine, 3-acrylamidopropyl phosphorylcholine, 4-acrylamidobutyl phosphorylcholine, 6-acrylamidohexyl phosphorylcholine, 10-acrylamidodecyl phosphorylcholine, and ω-(meth)acrylamide (poly)oxyethylene phosphorylcholin.

The regions (B) are adjacent to the regions (A) and have no cell adhesiveness or cell proliferation properties. If the regions (B) are regions which are adjacent to the regions (A) and have no cell proliferation properties, cell aggregates will be formed only in the regions (A) when cells are cultured, and it is possible to form a state in which there are no cells around some or all of the regions (A). In addition, the regions (B) preferably do not have cell adhesiveness as well as cell proliferation properties because they are suitable for uniformizing the size and shape of cell aggregates produced.

The shapes of the regions (B) are not limited except that the regions (B) are adjacent to the regions (A). However, a region (B) is preferably adjacent to 20% or more of the length of the boundary of a region (A), more preferably adjacent to 50% or more thereof, and still more preferably 80% or more thereof, and it is most preferable that the entire periphery of the region (A) be the region (B). In addition, it is preferable that the region (A) and the region (B) have a sea-island structure in which the region (A) has an island shape and the region (B) has a sea shape because these are suitable for increasing mass productivity of the cell culture substrate.

Although the area proportion of regions (A) and regions (B) is not particularly limited, the area of the regions (A) with respect to the total area of the regions (A) and the regions (B) is preferably 10% or more, more preferably 30% or more, still more preferably 50% or more, and most preferably 70% or more because this is suitable for increasing the number of cell aggregates that can be produced per unit area of the cell culture substrate. In addition, the area of the regions (B) with respect to the total area of the regions (A) and the regions (B) is preferably 20% or more, more preferably 40% or more, still more preferably 60% or more, and most preferably 80% or more because this is suitable for providing sufficient distances between the plurality of regions (A) and suppressing fusion of cell aggregates in the plurality of regions (A) to form an uneven shape.

The unevenness height at a boundary between a region (A) and a region (B) (the distance between the surface of the region (A) and the surface of the region (B) in the out-of-plane direction) is 1 to 500 nm. When the unevenness height is 500 nm or less, the number of dead cells captured by the unevenness can be suppressed, and the number of dead cells mixed into cell aggregates can be reduced. Accordingly, it is possible to increase the viability of cells in cell aggregates. In addition, if the unevenness height is 500 nm or less, air bubbles are less likely to adhere to the uneven portion. Accordingly, it is unnecessary to perform deaeration for removing the air bubbles or to repeat discharge and suction of a medium using a pipettor, thereby improving operability. When the unevenness height is 1 nm or more, living cells that have spontaneously moved (migrated) on the cell culture substrate are collected in the regions (A), and therefore it is possible to increase the viability of cells in cell aggregates. The unevenness height is more preferably 400 nm or less, still more preferably 100 nm or less, and most preferably 50 nm or less because this is suitable for increasing the viability of cells in cell aggregates formed.

The cell culture substrate may include a layer containing a living body-derived substance on its surface as necessary. The layer containing a living body-derived substance may be present on the entire surface of the cell culture substrate or may be present only on the surface of the regions (A). Living body-derived substances are not particularly limited, but examples thereof include matrigel, laminin, fibronectin, vitronectin, and collagen.

These living body-derived substances may be natural substances, may be artificially synthesized through a gene recombination technique or the like, or may be synthetic peptides, synthetic proteins, or the like obtained by chemically synthesizing fragments cut with restriction enzymes or substances equivalent to these living body-derived substances.

As matrigel, commercially available products such as Matrigel (manufactured by Corning Incorporated) and Geltrex (manufactured by Thermo Fisher Scientific Inc.) can be suitably used due to their easy availability.

The type of laminin is not particularly limited, and for example, laminin 511, laminin 521, laminin 511-E8 fragment, and the like can be used which have been reported to have high activity against α6β1 integrin expressed on the surface of human iPS cells. Laminin may be a natural substance, may be artificially synthesized through a gene recombination technique or the like, or may be a synthetic peptide or a synthetic protein obtained by chemically synthesizing a substance equivalent to laminin. A commercially available product such as iMatrix-511 (manufactured by Nippi Incorporated) or the like can be suitably used due to its easy availability.

Vitronectin may be a natural substance, may be artificially synthesized through a gene recombination technique or the like, or may be a synthetic peptide or a synthetic protein obtained by chemically synthesizing a substance equivalent to vitronectin. Commercially available products such as Vitronectin (manufactured by Wako Pure Chemical Industries, Ltd.) derived from human plasma, Synthemax (manufactured by Corning Incorporated), and Vitronectin (VTN-N) (manufactured by Thermo Fisher Scientific Inc.) can be suitably used due to their easy availability.

Fibronectin may be a natural substance, may be artificially synthesized through a gene recombination technique or the like, or may be a synthetic peptide or a synthetic protein obtained by chemically synthesizing a substance equivalent to fibronectin. Commercially available products such as Fibronectin Solution (manufactured by Wako Pure Chemical Industries, Ltd.) derived from human plasma and Retronectin (manufactured by Takara Bio Inc.) can be suitably used due to their easy availability.

The type of collagen is not particularly limited, and for example, type I collagen or type IV collagen can be used. Collagen may be a natural substance, may be artificially synthesized through a gene recombination technique or the like, or may be a synthetic peptide obtained by chemically synthesizing a substance equivalent to collagen. Commercially available products such as Human Collagen I (manufactured by Corning Incorporated) and Human Collagen IV (manufactured by Corning Incorporated) can be suitably used due to their easy availability.

From the viewpoints of being able to suppress denaturation of a living body-derived substance and to increase cell proliferation properties, the living body-derived substance is preferably immobilized on the cell culture substrate through a non-covalent bond. Here, the term "non-covalent bonds" indicates bonding forces, such as an electrostatic interaction, a water-insoluble interaction, a hydrogen bond, a π-π interaction, a dipole-dipole interaction, a London dispersion force, and other van der Waals interactions, other than covalent bonds derived from intermolecular forces. The immobilization of a living body-derived substance on a block copolymer may be performed through a single bonding force or a combination of a plurality of bonding forces.

The method for immobilizing a living body-derived substance is not particularly limited, and for example, a method for applying a living body-derived substance solution to a cell culture substrate for a predetermined period of time for immobilization or a method for adding a living body-derived substance to a culture liquid during culture of cells to adsorb the living body-derived substance onto the cell culture substrate for immobilization can be suitably used.

The cell culture substrate according to the present embodiment may be provided with a structure for partitioning each cell aggregate through providing a partition plate (for example, a plate having through-holes with a cross-sectional area of 0.05 to 100 cm² in an in-plane direction) on the substrate as necessary.

The cell culture substrate according to the present embodiment may be sterilized. The sterilization method is not particularly limited, but high pressure steam sterilization, UV sterilization, γ-ray sterilization, ethylene oxide gas sterilization, and the like can be used. From the viewpoint of suppressing denaturation of a block copolymer, high pressure steam sterilization, UV sterilization, or ethylene oxide gas sterilization are preferable. From the viewpoint of reducing deformation of the substrate, UV sterilization or ethylene oxide gas sterilization is more preferable. From the viewpoint of excellent mass productivity, ethylene oxide gas sterilization is preferable.

Cells to be cultured using the cell culture substrate according to the present embodiment are not particularly limited as long as they can adhere to the surface of the substrate before being simulated due to temperature drop. Examples thereof include not only various established cells such as Chinese hamster ovary-derived CHO cells, mouse connective tissue L929, human fetal kidney-derived HEK293 cells, or human cervical cancer-derived HeLa cells but also epithelial cells or endothelial cells that constitute various tissues and organs in a living body, skeletal muscle cells, smooth muscle cells, and cardiac muscle cells that exhibit contractility, neuron cells, glial cells, and fibroblasts that constitute the nervous system, hepatic parenchymal cells, hepatic non-parenchymal cells, and adipocytes that are involved in metabolism of a living body, differentiable cells such as mesenchymal stem cells, bone marrow cells, and stem cells in various tissues such as Muse cells, differentiated pluripotent stem cells (pluripotent stem cells) such as ES cells and iPS cells, and cells induced to differentiate therefrom. From the viewpoints of proliferation properties and peeling properties of cells in the cell culture substrate according to one embodiment, stem cells or pluripotent stem cells are preferable, mesenchymal stem cells or pluripotent stem cells are more preferable, pluripotent stem cells are still more preferable, and iPS cells are most preferable.

The cell culture substrate according to the present embodiment can be suitably used for inducing differentiation from pluripotent stem cells to three germ layer cells as described in examples to be described below. The cell culture substrate according to the present embodiment can also be suitably used for inducing differentiation from pluripotent stem cells to intestinal epithelium cells. Accordingly, the cell culture substrate according to the present embodiment may be used for inducing differentiation from pluripotent stem cells to three germ layer cells or may be used for inducing differentiation from pluripotent stem cells to intestinal epithelium cells.

The cell culture substrate according to the present embodiment can be produced through, for example, a production method including Step (1), Step (2), and Step (3) below. This production method is excellent in mass productivity. In Step (3), a corona treatment or a UV treatment can be performed instead of a plasma treatment.

Step (1) is a step of coating at least a part of the surface of the substrate with a composition containing a aUV-reactive hydrophilic polymer to form a layer containing the hydrophilic polymer.

Step (2) is a step of irradiating the layer containing the hydrophilic polymer with UV light to immobilize the layer containing the hydrophilic polymer on the surface of the substrate through a chemical reaction.

Step (3) is a step of subjecting a part of the surface of the immobilized layer containing the hydrophilic polymer to a plasma treatment to form regions (A) in the plasma-treated portions.

The cell culture substrate according to the present embodiment can also be produced through a production method including Step (1'), Step (2'), Step (3'), and Step (4') described below.

Step (1') is a step of using a substrate made of a polymer containing an alicyclic hydrocarbon group or an aromatic hydrocarbon group in a repeating unit and subjecting the surface of the substrate to a plasma treatment to form the above-described regions (A) in the plasma-treated portions.

Step (2') is a step of coating at least a part of the surface of the substrate with a composition containing a UV-reactive hydrophilic polymer to form a layer containing the above-described hydrophilic polymer.

Step (3') is a step of irradiating a part of the layer containing the above-described hydrophilic polymer with UV light to immobilize the part of the layer containing the above-described hydrophilic polymer on the surface of the above-described substrate.

Step (4') is a step of washing the above-described hydrophilic polymer with a solvent to dissolve the hydrophilic polymer that have not been immobilized on the surface and remove them from the surface of the substrate.

The production method according to the present embodiment may further include Step (4) below as necessary in addition to Step (1), Step (2), and Step (3), or Step (1'), Step (2'), Step (3'), and Step (4').

Step (4) is a step of bonding a plate having through-holes with a cross-sectional area of 0.05 to 100 cm² in an in-plane direction to the substrate on a surface side coated with the layer containing the hydrophilic polymer of the substrate after Step (3) or (4').

In addition, the production method according to one embodiment may further include Step (5) below after Step (3) or (4'). In a case of carrying out Step (4), Step (5) is preferably carried out after Step (3) or (4') and before Step (4).

Step (5) is a step of coating the plasma-treated surface of the layer containing the hydrophilic polymer with a composition containing a temperature-responsive polymer to form a layer containing the temperature-responsive polymer after Step (3) or (4').

In Step (1), at least a part of the surface of the substrate is coated with a composition containing a UV-reactive hydrophilic polymer to form a layer containing the hydrophilic polymer. By forming the layer containing the hydrophilic polymer, the layer can be made to have neither cell adhesiveness nor cell proliferation properties. The method for forming the layer containing the hydrophilic polymer is not particularly limited, and examples thereof include a method for coating at least a part of the surface of the substrate with a composition containing the hydrophilic polymer to form the layer. As the method for applying the composition containing the hydrophilic polymer, for example, generally known various methods such as coating, brush coating, dip coating, spin coating, bar coating, flow coating, spray coating, roll coating, air knife coating, blade coating, gravure coating, microgravure coating, and slot-die coating can be used.

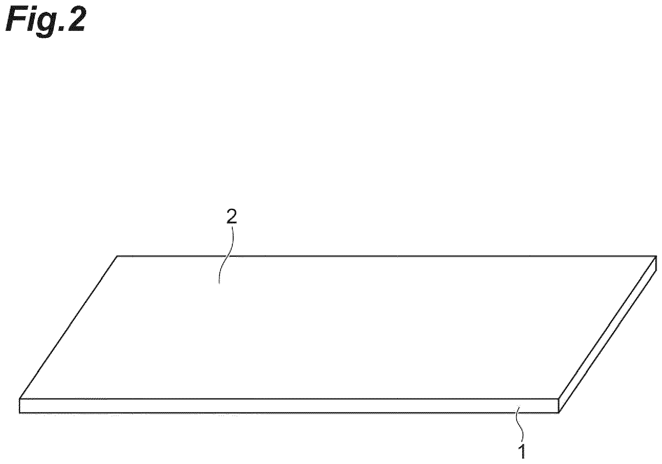

FIG. 2 is a schematic diagram (perspective view) of a substrate having a layer containing a hydrophilic polymer formed on its surface after Step (1). In FIG. 2, a layer 2 containing a hydrophilic polymer is formed on one entire surface of the substrate 1.

In Step (2), the layer 2 containing the hydrophilic polymer is irradiated with UV light to immobilize the layer containing the hydrophilic polymer on the surface of the substrate. By irradiating the UV-reactive hydrophilic polymer with UV light, a chemical reaction occurs between the hydrophilic polymer or between the substrate and the hydrophilic polymer, and the layer containing the hydrophilic polymer is immobilized on the surface of the substrate. By immobilizing the layer containing the hydrophilic polymer on the surface of the substrate, when a composition containing a temperature-responsive polymer is applied in Step (5) to be described below, the layer containing the temperature-responsive polymer can be formed without deforming the layer containing the hydrophilic polymer. In addition, by immobilizing the layer containing the hydrophilic polymer on the surface of the substrate, the shape of the regions (A) formed in a part of the surface of the layer containing the hydrophilic polymer in Step (3) to be described below can be maintained as well.

In Step (3), a part of the surface of the immobilized layer containing the hydrophilic polymer is subjected to a plasma treatment to form regions (A) in the plasma-treated portions. A suitable plasma treatment method can be appropriately adjusted depending on the type and layer thickness of the hydrophilic polymer used, but the plasma irradiation time is preferably 5 seconds to 30 minutes, more preferably 5 seconds to 10 minutes, particularly preferably 10 seconds to 5 minutes, and most preferably 15 seconds to 1 minute. In addition, a method for patterning the regions (A) is not particularly limited, but examples thereof include a method for performing a plasma treatment in a state in which the surface is covered with a metal mask, a silicone mask, a surface protection film, or the like to subject a desired portion (unmasked portion) to a plasma treatment.

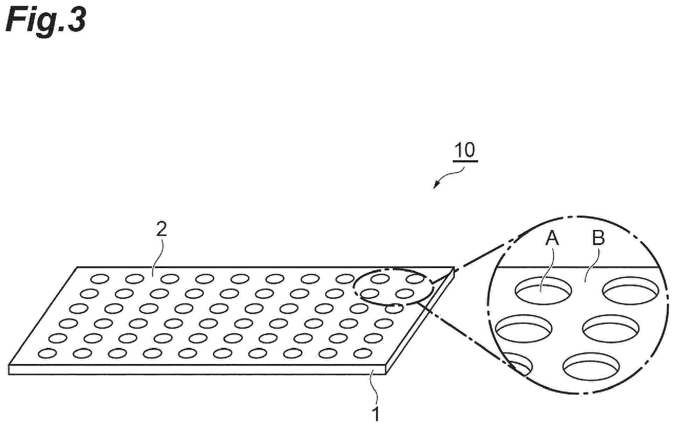

FIG. 3 is a schematic diagram (perspective view) of the cell culture substrate after Step (3). In the cell culture substrate 10 shown in FIG. 3, the circular regions (each of which being the region indicated by A; the area of the region being, for example, 0.001 to 5 mm²) arranged at equal intervals correspond to the plasma-treated portions. Since the surface of the layer 2 containing the hydrophilic polymer is modified through a plasma treatment or the like, the regions indicated by A have cell adhesiveness and cell proliferation properties. In addition, the region (region indicated by B) other than the circular regions corresponds to a portion which has not been subjected to a plasma treatment because it is protected by, for example, a metal mask or the like. Since the region indicated by B is a surface of the layer 2 containing the hydrophilic polymer, it does not have cell adhesiveness or cell proliferation properties.

In Step (1'), a polymer substrate containing an alicyclic hydrocarbon group or an aromatic hydrocarbon group in a repeating unit is used and the surface of the substrate is subjected to a plasma treatment to form the above-described regions (A) in the plasma-treated portions. By performing a plasma treatment on the surface of the polymer substrate containing an alicyclic hydrocarbon group or an aromatic hydrocarbon group, the surface of the substrate can be changed into a structure suitable for culturing cells. As plasma treatment conditions, air, nitrogen, or oxygen is used as an introduction gas, and the treatment is performed, at a gas pressure of 1 to 30 pascals, preferably for 1 second to 10 minutes, more preferably 1 second to 5 minutes, and particularly preferably 1 second to 1 minutes. Examples of alicyclic hydrocarbon groups include a cycloalkyl group, and more specific examples thereof include a cyclopropyl group, a cyclobutyl group, a cyclopentyl group, a cyclohexyl group, a cycloheptyl group, a cyclooctyl group, and a cyclononyl group. An aromatic hydrocarbon group means a group obtained by removing one or more hydrogen atoms directly bonded to carbon atoms constituting a ring from an aromatic hydrocarbon. Examples of aromatic hydrocarbon groups include a group obtained by removing one or more hydrogen atoms directly bonded to carbon atoms constituting a ring from a monocyclic aromatic hydrocarbon such as benzene and a polycyclic aromatic hydrocarbon such as naphthalene.

In addition, instead of Step (1') above, the surface of the substrate may be coated with a component suitable for culturing cells. As the component suitable for culturing cells, a polymer material such as polycarboxystyrene can be suitably used.

In Step (2'), at least a part of the surface of the substrate is coated with a composition containing a UV-reactive hydrophilic polymer to form a layer containing the above-described hydrophilic polymer. As the coating method, the same method as in Step (1) above can be suitably used.

In Step (3'), a part of the layer containing the above-described hydrophilic polymer is irradiated with UV light to immobilize the part of the layer containing the above-described hydrophilic polymer on the surface of the above-described substrate. By irradiating a partial region of the layer with UV light, only the UV-irradiated region of the layer containing the hydrophilic polymer is immobilized on the surface of the substrate. Examples of methods for irradiating a part of the layer containing the hydrophilic polymer with UV light include a method for placing a quartz mask or the like with a non-UV transmittable region formed through chromium deposition or the like in a desired pattern on the substrate to perform UV irradiation therefrom.

In Step (4'), the above-described hydrophilic polymer are washed with a solvent to dissolve the hydrophilic polymer that has not been immobilized on the surface and remove them from the surface of the substrate. By removing the hydrophilic polymer which has not been immobilized, the surface of the substrate can be exposed and the regions (A) can be formed. The solvent used in Step (4') preferably contains water and an alcohol because this is suitable for removing a compound by-produced trough a self-reaction of the UV-reactive hydrophilic polymer, for example, a compound containing nitrene derived from an azide group. By washing with a mixed solvent of water and an alcohol, the hydrophilic polymer-derived component remaining in the regions (A) can be reduced and the cell adhesiveness and cell proliferation properties of the regions (A) can be increased. As the above-described alcohol, lower alcohols such as methanol, ethanol, 2-propanol, t-butanol, isobutanol, pentanol, and hexanol are preferable, and methanol or ethanol is more preferable. In addition, the content of the above-described alcohol is preferably 50% to 95%, more preferably 50% to 90%, particularly preferably 60% to 90%, and most preferably 70% to 90%.

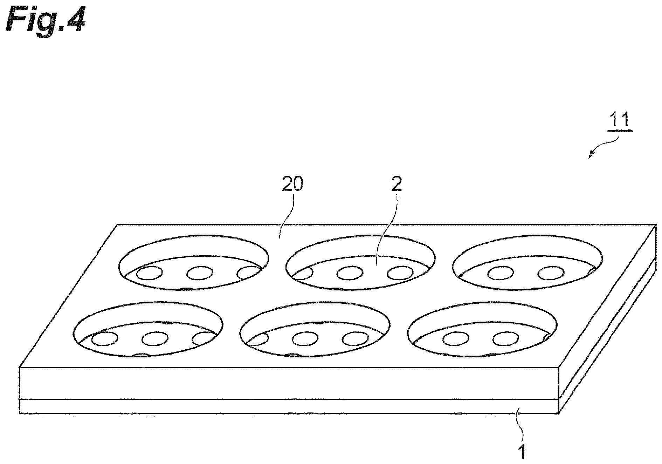

In Step (4), a plate having through-holes with a cross-sectional area of 0.05 to 100 cm² in an in-plane direction is bonded to the substrate on a surface side coated with the layer containing the hydrophilic polymer of the substrate after Step (3) or (4'). By bonding the plate having through-holes with a cross-sectional area of 0.05 to 100 cm² in an in-plane direction to the substrate, a plate having a space for a medium can be produced with high mass productivity. FIG. 4 is a schematic diagram (perspective view) of the cell culture substrate after Step (4). A cell culture substrate 11 shown in FIG. 4 is obtained, for example, by bonding a partition plate 20 (plate having through-holes with a cross-sectional area of 0.05 to 100 cm² in an in-plane direction) to the cell culture substrate 10 shown in FIG. 3.

In Step (5), the plasma-treated surface of the layer containing the hydrophilic polymer is coated with a composition containing a temperature-responsive polymer to form a layer containing the temperature-responsive polymer after Step (3) or (4'). At this time, formation of the layer containing the temperature-responsive polymer with a layer thickness of 100 nm or less is suitable for facilitating coating of the surface of the regions (A) formed in Step (3) or (4') with molecular chains of the temperature-responsive polymer in a sparse state and for imparting temperature responsiveness while maintaining the functions (including the cell adhesiveness and cell proliferation properties) of the regions (A). In addition, setting the layer thickness of the layer containing the temperature-responsive polymer to 1 nm or more is suitable because sufficient temperature responsiveness can be imparted and a cell culture substrate that enables rapid formation of cell aggregates can be produced.

As a method for applying a composition containing a temperature-responsive polymer, the same method as the above-described method for applying a composition containing a hydrophilic polymer can be suitably used.

As the method for applying a composition containing a atemperature-responsive polymer, it is also preferable to coat the entire surface of the cell culture substrate with a temperature-responsive substance. When applying a composition containing a temperature-responsive polymer, the mass productivity of the cell culture substrate can be increased through application using a commonly used application method without performing patterning. In addition, by coating the entire surface of the cell culture substrate with the composition containing a temperature-responsive polymer, the temperature responsiveness is imparted to the regions (A), and the regions (B) are also coated with the temperature-responsive polymer. By coating the regions (B) with the temperature-responsive polymer, the cell adhesiveness of the regions (B) can be reduced.

The present invention also relates to a method for inducing differentiation of pluripotent stem cells.

The differentiation induction method according to the present embodiment includes Steps (i) to (iii) below. The differentiation induction method according to the present embodiment is suitably used for inducing differentiation from pluripotent stem cells to three germ layer cells.

Step (i) is a step of seeding the cell culture substrate according to the present invention with pluripotent stem cells.

Step (ii) is a step of culturing the seeded pluripotent stem cells to form hemispherical cell aggregates with a height-to-diameter ratio of 0.2 to 0.8.

Step (iii) is a step of inducing differentiation of the above-described cell aggregates to form cell aggregates of three germ layer cells.

Step (i) is a step in which the above-described cell culture substrate according to the present invention is used to seed pluripotent stem cells therein. The expression "seeding cells" indicates that a cell-dispersed medium (hereinafter referred to as a "cell suspension") is, for example, applied to or injected into the cell culture substrate to bring the cell suspension into contact with the cell culture substrate. A cell culture substrate having a cell-proliferative region can be used to culture cells in Step (ii) to be described below. In a case where a cell culture substrate does not have a cell-proliferative region, cells cannot be cultured in Step (ii).

In Step (i) above, the culture is carried out under conditions effective for maintaining undifferentiation of pluripotent stem cells. The conditions effective for maintaining the undifferentiation are not particularly limited. For example, the density of pluripotent stem cells at the start of culture should be within preferred ranges described below as the cell density during seeding, and the culture is performed in the presence of an appropriate liquid medium. As media effective for maintaining undifferentiation of pluripotent stem cells, it is possible suitably use media to which one or more factors, such as insulin, transferrin, selenium, ascorbic acid, sodium hydrogen carbonate, basic fibroblast growth factor, transforming growth factor β (TGFβ), CCL2, activins, and 2-mercaptomethanol, known to maintain undifferentiation of pluripotent stem cells are added. Since these are particularly suitable for maintaining undifferentiation of pluripotent stem cells, media containing insulin, transferrin, selenium, ascorbic acid, sodium hydrogen carbonate, basic fibroblast growth factor, or transforming growth factor β (TGFβ) are more preferably used, and media to which basic fibroblast growth factor is added is most preferably used.

The types of media to which the above-described basic fibroblast growth factor is added are not particularly limited, but examples of commercially available products thereof include DMEM (manufactured by Sigma-Aldrich Co. LLC.), Ham's F12 (manufactured by Sigma-Aldrich Co. LLC.), D-MEM/Ham's F12 (manufactured by Sigma-Aldrich Co. LLC.), Primate ES Cell Medium (manufactured by Reprocell Inc.), StemFit AK02N (manufactured by Ajinomoto Co., Inc.), StemFit AK03 (manufactured by Ajinomoto Co., Inc.), mTeSR1 (manufactured by STEMCELL Technologies), TeSR-E8 (manufactured by STEMCELL technologies), ReproNative (manufactured by Reprocell Inc.), ReproXF (manufactured by Reprocell Inc.), ReproFF (manufactured by Reprocell Inc.), ReproFF2, (manufactured by Reprocell Inc.), NutriStem (manufactured by Biological Industries), iSTEM (manufactured by Takara Bio Inc.), GS2-M (manufactured by Takara Bio Inc.), and hPSC Growth Medium DXF (manufactured by PromoCell GmbH). Since these are suitable for maintaining undifferentiated states of pluripotent stem cells, Primate ES Cell Medium (manufactured by Reprocell Inc.), StemFit AK02N (manufactured by Ajinomoto Co., Inc.), or StemFit AK03 (manufactured by Ajinomoto Co., Inc.) is preferable, StemFit AK02N (manufactured by Ajinomoto Co., Inc.) or StemFit AK03 (manufactured by Ajinomoto Co., Inc.) is more preferable, and StemFit AK02N (manufactured by Ajinomoto Co., Inc.) is particularly preferable.

In Step (i) above, the method for seeding pluripotent stem cells is not particularly limited, but it can be performed, for example, by injecting a cell suspension into the cell culture substrate. The cell density during seeding is not particularly limited, but is preferably 1.0×10² to 1.0×10⁶ cells/cm², more preferably 5.0×10² to 5.0×10⁵ cells/cm², still more preferably 1.0×10³ to 2.0×10⁵ cells/cm², and most preferably 1.2×10³ to 1.0×10⁵ cells/cm² so that pluripotent stem cells can be maintained and proliferated.

A medium obtained by further adding a Rho-binding kinase inhibitor to a medium to which the above-described basic fibroblast growth factor is added is preferably used as a medium used in Step (i) above because this is suitable for maintaining the viability of pluripotent stem cells. In a case of particularly using human pluripotent stem cells, if a Rho-binding kinase inhibitor is added in a state where the cell density of human pluripotent stem cells is low, it may be effective in maintaining the viability of human pluripotent stem cells. As the Rho-binding kinase inhibitor, (R)-(+)-trans-N-(4-pyridyl)-4-(1-aminoethyl)-cyclohexanecarbo xamide·2HCl·H2O (Y-27632 manufactured by Wako Pure Chemical Industries, Ltd.) or 1-(5-Isoquinolinesulfonyl)homopiperazine hydrochloride (HA1077 manufactured by Wako Pure Chemical Industries, Ltd.) can be used, for example. The concentration of a Rho-binding kinase inhibitor added to a medium is within a range effective in maintaining the viability of human pluripotent stem cells and a range that does not affect an undifferentiated state of human pluripotent stem cells, and is preferably 1 µM to 50 µM, more preferably 3 µM to 20 µM, still more preferably 5 µM to 15 µM, and most preferably 8 µM to 12 µM.

Shortly after starting Step (i) above, the pluripotent stem cells begin to adhere to the cell culture substrate.

In Step (ii), the seeded pluripotent stem cells are cultured to form hemispherical cell aggregates with a height-to-diameter ratio of 0.2 to 0.8. The "height" may be, for example, the maximum value of the diameter of the cells in the out-of-plane direction of the cell culture substrate. The "diameter" may be, for example, the maximum value of the diameter of the cells in the in-plane direction of the cell culture substrate.

The culture temperature in Step (ii) is preferably 30°C to 42°C, more preferably 32°C to 40°C, still more preferably 36°C to 38°C, and most preferably 37°C because this is suitable for maintaining proliferation ability, physiological activity, or function of the pluripotent stem cells.

It is preferable to perform the first medium exchange 22 to 26 hours after starting Step (ii). It is preferable to perform second medium exchange 48 to 72 hours later, and thereafter, to perform medium exchange every 24 to 48 hours. During this time, pluripotent stem cells proliferate and form flat cell aggregations called colonies. Colonies are in a state in which cells two-dimensionally adhere to the cell culture substrate. The culture is continued until the sizes of the colonies reach the sizes of the regions (A), and by further continuing the culture, three-dimensional cell aggregates are formed. The shapes of the three-dimensional cell aggregates are preferably hemispherical with a height-to-diameter ratio of 0.2 to 0.8. The height-to-diameter ratio is determined by observing a plurality of cell aggregates using a microscope and calculating and averaging the values. By forming hemispherical cell aggregates with a height-to-diameter ratio of 0.2 to 0.8, inducing differentiation from pluripotent stem cells to three germ layer cells can be efficiently performed. In addition, the height-to-diameter ratio is more preferably 0.3 to 0.7 and still more preferably 0.4 to 0.6 because this is suitable for increasing the efficiency of differentiation induction into three germ layer cells.

In Step (iii), differentiation of the above-described cell aggregates (the cell aggregates of the pluripotent stem cells) is induced to form cell aggregates of three germ layer cells. Step (iii) may be performed after Step (ii) or may be performed in parallel with Step (ii). That is, after the seeded pluripotent stem cells adhere to the cell culture substrate, Step (ii) and Step (iii) may be started immediately to perform differentiation induction while forming cell aggregates. Three germ layer cells refer to any of endoderm cells, mesoderm cells, or ectoderm cells. In Step (iii), cells are cultured in a medium containing a differentiation-inducing factor.

The differentiation-inducing factor in Step (iii) preferably contains an endoderm-inducing factor because this is suitable for increasing the efficiency of differentiation induction into endoderm cells. Since it is suitable for increasing the efficiency of differentiation induction into embryoid bodies, the endoderm-inducing factor is preferably single or multiple differentiation-inducing factors selected from the group consisting of a Wnt protein, a bone morphogenetic protein (BMP), an insulin-like growth factor, and an activin, and particularly preferably contains any of Wnt3a, BMP4, IGFI, or Activin A.

The differentiation-inducing factor in Step (iii) preferably contains a mesoderm-inducing factor because this is suitable for increasing the efficiency of differentiation induction into mesoderm cells. Since it is suitable for increasing the efficiency of differentiation induction into embryoid bodies, the mesoderm-inducing factor is preferably single or multiple differentiation-inducing factors selected from the group consisting of a GSK3β inhibitor, a bone morphogenetic protein (BMP), and an activin, and particularly preferably contains any of Activin A, CHIR99021 (GSK3β inhibitor), or BMP4.

The differentiation-inducing factor in Step (iii) preferably contains an ectoderm-inducing factor because this is suitable for increasing the efficiency of differentiation induction into ectoderm cells. The above-described ectoderm-inducing factor preferably contains any of Noggin (BMP inhibitor), dorsomorphin (BMP inhibitor), SB431542 (TGF-β inhibitor), or an activin inhibitor and more preferably contains a BMP inhibitor or a TGF-β inhibitor.

The concentration of a differentiation-inducing factor added to a medium is not particularly limited but is preferably 500 to 1,000 ng/mL, more preferably 100 to 500 ng/mL, and most preferably 100 ng/mL because this is suitable for increasing the efficiency of differentiation induction into three germ layer cells.

The medium containing a differentiation-inducing factor is preferably exchanged every 24 hours during culture to allow differentiation induction to progress sufficiently. By exchanging the medium within 24 hours, it is possible to prevent a shortage of a differentiation-inducing factor in the medium and to uniformly differentiate all pluripotent stem cells.

The method for inducing differentiation of pluripotent stem cells according to the present embodiment may further have Step (iv) below. The differentiation induction method in the case of including Step (iv) is suitably used for inducing differentiation from pluripotent stem cells to intestinal epithelial cells.

Step (iv) is a step of culturing the cell aggregates in a medium containing at least one differentiation-inducing factor selected from the group consisting of a Wnt protein, a bone morphogenetic protein (BMP), an insulin-like growth factor, and an activin to express markers possessed by intestinal epithelium cells.

Intestinal epithelium cells preferably have any one or a plurality of cells of intestinal cells, goblet cells, enteroendocrine cells, Paneth cells, and intestinal epithelium step cells and more preferably contain all of intestinal cells, goblet cells, enteroendocrine cells, Paneth cells, and intestinal epithelium stem cells. Intestinal epithelium cells have unique markers. Examples thereof include CDX2 and VIL1 as intestinal cell markers, MUC2 as a goblet cell marker, CGA as an enteroendocrine cell marker, DEFA6 as a Paneth cell marker, and LGR5 as an intestinal epithelium step cell marker.

Since the cell culture substrate according to the present invention does not have an uneven structure with a high height in the out-of-plane direction on its surface, air bubbles are difficult to adhere to the surface of the cell culture substrate when a medium is brought into contact with the cell culture substrate. The number of air bubbles adhering to the cell culture substrate according to the present invention based on the number of regions (A) is, for example, preferably 10% or less, more preferably 5% or less, still more preferably 3% or less, and most preferably 1% or less.

Cell aggregates formed using the cell culture substrate according to the present invention have a higher cell viability due to spontaneous aggregation of adhered cells compared to formation of cell aggregates using a fine unevenness structure in the past. For example, in a case where cells with a viability of 50% are used, the viability of cells in cell aggregates formed is preferably 70% or more, more preferably 80% or more, still more preferably 85% or more, and most preferably 90% or more.

### Examples

Hereinafter, the present invention will be described in more detail based on examples. However, the present invention is not limited to the following examples. Unless otherwise specified, commercially available reagents were used.

### <Measurement of area of regions (A)>

A laser microscope (manufactured by Keyence Corporation, product name of VK-X200) was used to obtain an image of the surface of a cell culture substrate. The obtained image was used to determine the areas of regions (A) at 20 points on analysis software VK-X Viewer, and these areas were averaged to obtain the area of a region (A).

### <Measurement of unevenness height at boundary between region (A) and region (B)>

The unevenness height at a boundary was measured by measuring the thickness of the cell culture substrate in the out-of-plane direction using a laser microscope (manufactured by Keyence Corporation, product name of VK-X200) in laser scanning mode.

### <Evaluation of adhesion of air bubbles>

StemFit AK02N medium (manufactured by Ajinomoto Co., Inc.) was added to a cell culture substrate, and the presence or absence of adhesion of air bubbles was confirmed through observation with a microscope.

### <Evaluation of cell aggregates>

### (Culture of cell aggregates)

0.2 mL/cm² of StemFit AK02N medium (manufactured by Ajinomoto Co., Inc.) was added to a cell culture substrate, and an iMatrix-511 solution (manufactured by Nippi Inc.) was added thereto at a concentration of 2.5 µL/mL. Human iPS cells 201B7 strain which were adjusted to have a ratio of viable cells to all cells (cell viability) of 50% by mixing dead cells were used to perform seeding at 15,000 cells (total number of living cells and dead cells)/cm² and perform culture in an environment of 37°C and a CO₂ concentration of 5%. In addition, Y-27632 (manufactured by Wako Pure Chemical Industries, Ltd.) (concentration of 10 µM) was added to the medium until 24 hours after the cells were seeded. The presence or absence of formation of cell aggregates was confirmed one or two days after the start of culture.

### (Detachment of cell aggregates due to cooling)

After confirming the formation of cell aggregates, detachment of the cell aggregates was confirmed through cooling at room temperature for 30 minutes.

### (Evaluation of cell viability)

After confirming the formation of cell aggregates, cells were peeled off and collected as single cells using a cell scraper after being treated with a TrypLE-EDTA solution (1:1 mixture of TrypLE select (manufactured by Thermo Fisher Scientific Inc.) and a 0.5 mM EDTA solution (manufactured by Invitrogen)) and stained with trypan blue to measure the cell viability.

### <Measurement of thickness of hydrophilic polymer and temperature-responsive polymer layers>

The thickness of polymer (hydrophilic polymer and temperature-responsive polymer) layers covered on a substrate was determined from the surface area of the substrate, the concentrate of the polymer in the solutions, and the volume of the solutions added dropwise onto the substrate by calculating the amount of the coating per unit area with the specific gravity of the polymer as 1.

### <Measurement of composition of temperature-responsive polymer>

The composition of the temperature-responsive polymer was determined through proton nuclear magnetic resonance spectroscopy (¹H-NMR) spectral analysis using a nuclear magnetic resonance measurement device (manufactured by JEOL Ltd., trade name of JNM-GSX400) or carbon nuclear magnetic resonance spectroscopy (¹³C-NMR) spectral analysis using a nuclear magnetic resonance measurement device (manufactured by Bruker, product name of AVANCE III HD500).

### <Measurement of molecular weight and molecular weight distribution of temperature-responsive polymer>

The weight average molecular weight (Mw), the number average molecular weight (Mn), and the molecular weight distribution (Mw/Mn) were measured through gel permeation chromatography (GPC). HLC-8320GPC manufactured by Tosoh Corporation was used as a GPC device, two TSKgel Super AWM-H columns manufactured by Tosoh Corporation were used, the column temperature was set to 40°C, and 1,1,1,3,3,3-hexafluoro-2-isopropanol containing 10 mM sodium trifluoroacetate or N,N-dimethylformamide containing 10 mM lithium bromide were used as eluents to perform the measurement. The measurement sample was prepared at 1.0 mg/mL to perform the measurement. For the molecular weight calibration curve, polymethyl methacrylate (manufactured by Polymer Laboratories Ltd.) with a known molecular weight was used.

### <Evaluation of autofluorescence intensities of substrates>

Substrates were irradiated with light beams respectively having excitation wavelengths of 350 nm, 488 nm, and 647 nm with an exposure time of 0.4 seconds, and fluorescence images were taken. RGB values of these images were obtained, an R value at an excitation wavelength of 350 nm, a G value at an excitation wavelength of 488 nm, and a B value at an excitation wavelength of 647 nm were defined as fluorescence intensities. The same measurement was performed on a 1.2 mm-thick polystyrene plate, and relative values to the value in the 1.2 mm-thick polystyrene plate were shown in Table 4 as fluorescence intensities of the substrates.

### [Example 1]

0.8 mL of a water-ethanol solution containing polyvinyl alcohol (BIOSURFINE (R)-AWP, manufactured by Toyo Gosei Co., Ltd.) having an azide group as a hydrophilic polymer at a solid content concentration of 0.06 wt% was added dropwise onto a polystyrene (PS) dish (substrate) with a diameter of 3.5 cm and dried under reduced pressure at room temperature, and then the hydrophilic polymer were cured through UV irradiation to form a hydrophilic polymer layer. A metal mask having a plurality of circular holes having a diameter of 0.2 mm was placed on the hydrophilic polymer layer, and a plasma treatment (under a gas pressure of 20 Pa with a conduction current of 20 mA for an irradiation time of 30 seconds) was performed from above the metal mask using plasma irradiation device (manufactured by Vacuum Device, trade name of Plasma Ion Bombarder PIB-20) to form regions (regions (A)) having cell adhesiveness and cell proliferation properties in the plasma-treated portion. In addition, regions (B) were formed in the portion masked with a metal mask.

The structure of the produced cell culture substrate and evaluation results are shown in Table 2. The produced cell culture substrate had a layer thickness of a hydrophilic polymer layer of 600 nm, an area of a region (A) of 0.03 mm², an unevenness height at a boundary between a region (A) and a region (B) of 32 nm.

Culture of the human iPS cells on this cell culture substrate was evaluated, and the formation of cell aggregates was confirmed two days after the start of culture. In visual assessment, the sizes of the formed cell aggregates were uniform. The height-to-diameter ratio of the cell aggregates was estimated to be 0.5. In addition, the viability of cells contained in the cell aggregates was 89%, which was a high cell viability. In addition, air bubbles had not adhered to the cell culture substrate during addition of a medium.

### [Example 2]

A cell culture substrate was produced in the same manner as in Example 1 except that the plasma irradiation time in Example 1 was changed to 10 minutes.

The structure of the produced cell culture substrate and evaluation results are shown in Table 2. The produced cell culture substrate had a layer thickness of a hydrophilic polymer layer of 600 nm, an area of a region (A) of 0.03 mm², an unevenness height at a boundary between a region (A) and a region (B) of 85 nm.

Culture of the human iPS cells on this cell culture substrate was evaluated, and the formation of cell aggregates was confirmed two days after the start of culture. In visual assessment, the sizes of the formed cell aggregates were uniform. The height-to-diameter ratio of the cell aggregates was estimated to be 0.5. In addition, the viability of cells contained in the cell aggregates was 91%, which was a high cell viability. In addition, air bubbles had not adhered to the cell culture substrate during addition of a medium.

### [Example 3]

A cell culture substrate was produced in the same manner as in Example 1 except that the gas pressure for a plasma treatment in Example 1 was changed to 13 Pa and the plasma irradiation time was changed to 20 minutes.

The structure of the produced cell culture substrate and evaluation results are shown in Table 2. The produced cell culture substrate had a layer thickness of a hydrophilic polymer layer of 600 nm, an area of a region (A) of 0.03 mm², an unevenness height at a boundary between a region (A) and a region (B) of 452 nm.

Culture of the human iPS cells on this cell culture substrate was evaluated, and the formation of cell aggregates was confirmed two days after the start of culture. In visual assessment, the sizes of the formed cell aggregates were uniform. The height-to-diameter ratio of the cell aggregates was estimated to be 0.5. In addition, the viability of cells contained in the cell aggregates was 78%, which was a high cell viability. In addition, air bubbles had not adhered to the cell culture substrate during addition of a medium.

### [Example 4]

0.08 mL of an ethanol solution containing a polymer (Lipidure-CM5210, manufactured by NOF Corporation) containing a phosphorylcholine group as a hydrophilic polymer at a solid content concentration of 0.01 wt% was added dropwise onto a polystyrene dish (substrate) with a diameter of 3.5 cm and dried under reduced pressure at room temperature to form a hydrophilic polymer layer. A metal mask having a plurality of circular holes having a diameter of 0.2 mm was placed on the hydrophilic polymer layer, and a plasma treatment (under a gas pressure of 20 Pa with a conduction current of 20 mA for an irradiation time of 30 seconds) was performed from above the metal mask using a plasma irradiation device (manufactured by Vacuum Device, trade name of Plasma Ion Bombarder PIB-20) to form regions (regions (A)) having cell adhesiveness and cell proliferation properties in the plasma-treated portion. In addition, regions (B) were formed in the portion masked with a metal mask.

The structure of the produced cell culture substrate and evaluation results are shown in Table 2. The produced cell culture substrate had a layer thickness of a hydrophilic polymer layer of 10 nm, an area of a region (A) of 0.03 mm², an unevenness height at a boundary between a region (A) and a region (B) of 23 nm.

Culture of the human iPS cells on this cell culture substrate was evaluated, and the formation of cell aggregates was confirmed two days after the start of culture. In visual assessment, the sizes of the formed cell aggregates were uniform. The height-to-diameter ratio of the cell aggregates was estimated to be 0.5. In addition, the viability of cells contained in the cell aggregates was 87%, which was a high cell viability. In addition, air bubbles had not adhered to the cell culture substrate during addition of a medium.

### [Example 5]

A cell culture substrate was produced in the same manner as in Example 4 except that the solid content concentration of the hydrophilic polymer was changed to 0.05 wt%.

The structure of the produced cell culture substrate and evaluation results are shown in Table 2. The produced cell culture substrate had a layer thickness of a hydrophilic polymer layer of 50 nm, an area of a region (A) of 0.03 mm², an unevenness height at a boundary between a region (A) and a region (B) of 21 nm.

Culture of the human iPS cells on this cell culture substrate was evaluated, and the formation of cell aggregates was confirmed two days after the start of culture. In visual assessment, the sizes of the formed cell aggregates were uniform. The height-to-diameter ratio of the cell aggregates was estimated to be 0.5. In addition, the viability of cells contained in the cell aggregates was 92%, which was a high cell viability. In addition, air bubbles had not adhered to the cell culture substrate during addition of a medium.

### [Example 6]

A cell culture substrate was produced in the same manner as in Example 1 except that the solid content concentration of the hydrophilic polymer was changed to 0.15 wt%.

The structure of the produced cell culture substrate and evaluation results are shown in Table 2. The produced cell culture substrate had a thickness of a hydrophilic polymer layer of 1500 nm, an area of a region (A) of 0.03 mm², an unevenness height at a boundary between a region (A) and a region (B) of 34 nm.

Culture of the human iPS cells on this cell culture substrate was evaluated, and the formation of cell aggregates was confirmed two days after the start of culture. In visual assessment, the sizes of the formed cell aggregates were uniform. The height-to-diameter ratio of the cell aggregates was estimated to be 0.5. In addition, the viability of cells contained in the cell aggregates was 85%, which was a high cell viability. In addition, air bubbles had not adhered to the cell culture substrate during addition of a medium.

### [Example 7]

A cell culture substrate was produced in the same manner as in Example 1 except that a polyethylene terephthalate (PET) film (Lumirror, manufactured by Toray Industries Inc.) (substrate) having a film thickness of 188 µm was used instead of the polystyrene (PS) dish having a diameter of 3.5 cm. Culture was performed by immobilizing cutouts of the produced cell culture substrate in a petri dish.

The structure of the produced cell culture substrate and evaluation results are shown in Table 2. The produced cell culture substrate had a thickness of a hydrophilic polymer layer of 600 nm, an area of a region (A) of 0.03 mm², an unevenness height at a boundary between a region (A) and a region (B) of 38 nm.

Culture of the human iPS cells on this cell culture substrate was evaluated, and the formation of cell aggregates was confirmed two days after the start of culture. In visual assessment, the sizes of the formed cell aggregates were uniform. The height-to-diameter ratio of the cell aggregates was estimated to be 0.5. In addition, the viability of cells contained in the cell aggregates was 84%, which was a high cell viability. In addition, air bubbles had not adhered to the cell culture substrate during addition of a medium.

### [Example 8]

A cell culture substrate was produced in the same manner as in Example 1 except that a metal mask having a plurality of circular holes with a diameter of 1.5 mm was used instead of the metal mask having a plurality of circular holes having a diameter of 0.2 mm.

The structure of the produced cell culture substrate and evaluation results are shown in Table 2. The produced cell culture substrate had a thickness of a hydrophilic polymer layer of 600 nm, an area of a region (A) of 1.76 mm², an unevenness height at a boundary between a region (A) and a region (B) of 35 nm.

Culture of the human iPS cells on this cell culture substrate was evaluated, and the formation of cell aggregates was confirmed two days after the start of culture. In visual assessment, the sizes of the formed cell aggregates were uniform. The height-to-diameter ratio of the cell aggregates was estimated to be 0.5. In addition, the viability of cells contained in the cell aggregates was 89%, which was a high cell viability. In addition, air bubbles had not adhered to the cell culture substrate during addition of a medium.

Using this cell culture substrate, the human iPS cells were induced to differentiate into intestinal epithelium cells. A 1% Vitronectin (VTN-N) Recombinant Human Protein solution (manufactured by Gibco) was added to the cell culture substrate at 1.0 mL/dish and allowed to stand for 1 hour at 25°C. After 1 hour, the 1% VTN solution was removed, StemFit AK02N (manufactured by Ajinomoto Co., Inc.) which was an undifferentiation-maintaining medium was added thereto at 2.0 mL/dish, the human iPS cells 201B7 strain were further seeded at 3,900 cells/cm² and cultured in an environment of 37°C and a CO₂ concentration of 5%. In addition, Y-27632 (manufactured by FUJIFILM Wako Pure Chemical Corporation) (concentration of 10 µM) was added to the medium until 24 hours after cell seeding. A plurality of circular embryoid bodies adhering to the cell culture substrate were confirmed through phase-contrast microscopic observation.

24 Hours after cell seeding, the following differentiation induction medium was added thereto at 2.0 mL/dish to start differentiation induction.

The composition of the medium inducing differentiation into intestinal epithelium cells is 85% KnockOut DMEM (manufactured by Thermo Fisher Scientific Inc.), 15% KnockOut Serum Replacement XenoFree (manufactured by Thermo Fisher Scientific Inc.), 0.1 mM non-essential amino acid (manufactured by Sigma-Aldrich Co. LLC.), 2 mM Gluta Max-I Supplement (manufactured by Thermo Fisher Scientific Inc.), 20 ng/mL basic fibroblast growth factor (bFGF, manufactured by PeproTech, Inc.), 50 µg/mL L(+)-ascorbic acid (FUJIFILM Wako Pure Chemical Corporation), 10 ng/mL heregulin-β-1 (FUJIFILM Wako Pure Chemical Corporation), 200 ng/mL Long (R) R3IGF-I (manufactured by Sigma-Aldrich Co. LLC.), and a 1% penicillin-streptomycin solution (×100) (FUJIFILM Wako Pure Chemical Corporation). The time point when the differentiation-inducing medium was added was defined as "day 0 of differentiation." Thereafter, the differentiation-inducing medium was exchanged every 72 hours of culture until "day 64 of differentiation."

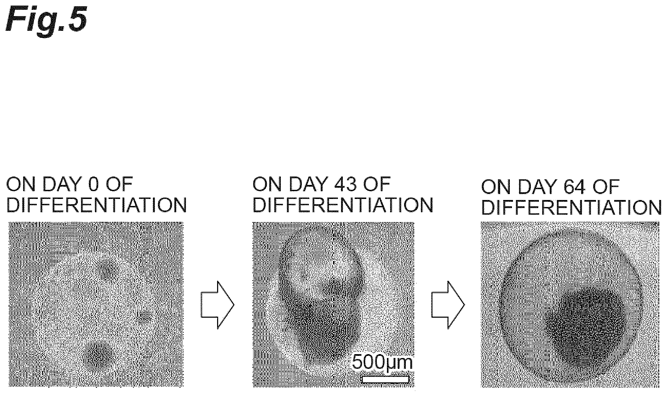

FIG. 5 shows phase-contrast microscopic images of cells on "day 0 of differentiation," "day 43 of differentiation," and "day 64 of differentiation." As shown in FIG. 5, cyst formation derived from the small intestine was confirmed on "day 43 of differentiation," and the cyst was peeled off from the cell culture substrate on "day 64 of differentiation."

On "day 64 of differentiation," the above-described differentiation-inducing medium in the dish was removed, and PBS(-) was added at 2.0 mL/dish for washing. After washing, a cell detachment solution was added to the dish at 1.0 mL/dish and allowed to stand for 5 minutes in an environment of 37°C and a CO₂ concentration of 5%. After allowing the cells to stand for a predetermined period of time, the cell detachment solution was removed, and a differentiation-inducing medium was added thereto at 1.0 mL/dish to disperse the cells. The obtained cell suspension was collected, and the number of cells was counted using a Luna Automatic Cell Counter (manufactured by Logos Biosystems).

To evaluate the induction of differentiation into intestinal epithelium cells, a housekeeping gene marker (GAPDH), an intestinal cell marker (CDX2), an absorptive epithelial marker (VIL1), and an intestinal stem cell marker (LGR5) were adopted, and relative expression levels of mRNA of the intestinal cell marker and the intestinal stem cell marker were quantitatively determined. Small intestine tissue (R1234226-50, BioChain Institute Inc.) from a healthy adult was used as a positive control.

The cells on "day 64 of differentiation" which were collected through the counting of the number of cells were fractionated into 1.5 mL sample tubes at 1.0×10° cells/tube. Centrifugation (room temperature, 800 × g, 5 minutes) was performed, and a supernatant was removed. RNeasy Plus Mini Kit (manufactured by QIAGEN) was used to extract RNA from the cells. The concentration of the extracted RNA was measured with a Qubit 4 fluorometer and adjusted to 1 µg/6 µL with RNase-free water (manufactured by Takara Bio Inc.). ReverTra Ace qRT Master Mix with gDNA Remover (manufactured by Toyobo Co., Ltd.) was used for a reverse transcription reaction. For 1 µL of 5-fold diluted cDNA (equivalent to 1/100 of a reverse transcription product), an oligo-dT primer, primers (manufactured by Integrated DNA Technologies, Inc.) having base sequences shown in Table 1 below, and THUNDERBIRD Probe qPCR Mix (manufactured by Toyobo Co., Ltd.) were used to perform real-time PCR analysis using QuantStudio 3 Real-Time PCR System (manufactured by Thermo Fisher Scientific Inc.). As a result, it was confirmed that intestinal tissue-derived CDX2, VIL1, and LGR5 were expressed in the cells on "day 64 of differentiation."

**[Table 1]**

| Primer | SEQ ID NO | Sequence (5'→3') |
|---|---|---|
| GAPDH | SEQ ID NO: 1 | ACATCGCTCAGACACCATGTGTAGTTGAGGTCAATGAAGGG |
| CDX2 | SEQ ID NO: 2 | GCTGGAGAAGGAGTTTCACTACCTTTGCTCTGCGGTTCTGA |
| VIL1 | SEQ ID NO: 3 | TTGCCACAATTCCCTGAGATGATGTTGAGAGAGCCTTTGACT |
| LGR5 | SEQ ID NO: 4 | GGAATGTTTCAGGCTCAAGATGTCAAGCAGGTGTTCACAGG |

### [Example 9]

0.08 mL of an ethanol solution containing a polymer (Lipidure-CM5210, manufactured by NOF Corporation) containing a phosphorylcholine group and a UV-reactive functional group as a hydrophilic polymer at a solid content concentration of 0.05 wt% was added dropwise onto a polystyrene dish (substrate) with a diameter of 3.5 cm and dried under reduced pressure at room temperature to form a hydrophilic polymer layer. A metal mask having a plurality of circular holes having a diameter of 0.2 mm was placed on the hydrophilic polymer layer, and a plasma treatment (under a gas pressure of 20 Pa with a conduction current of 20 mA for an irradiation time of 30 seconds) was performed from above the metal mask using a plasma irradiation device (manufactured by Vacuum Device, trade name of Plasma Ion Bombarder PIB-20) to form regions (regions (A)) having cell adhesiveness and cell proliferation properties in the plasma-treated portion. In addition, regions (B) were formed in the portion masked with a metal mask.

The structure of the produced cell culture substrate and evaluation results are shown in Table 2. The produced cell culture substrate had a layer thickness of a hydrophilic polymer layer of 50 nm, an area of a region (A) of 0.03 mm², an unevenness height at a boundary between a region (A) and a region (B) of 33 nm.

Culture of the human iPS cells on this cell culture substrate was evaluated, and the formation of cell aggregates was confirmed two days after the start of culture. In visual assessment, the sizes of the formed cell aggregates were uniform. The height-to-diameter ratio of the cell aggregates was estimated to be 0.5. In addition, the viability of cells contained in the cell aggregates was 86%, which was a high cell viability. In addition, air bubbles had not adhered to the cell culture substrate during addition of a medium.

### [Example 10]

A cell culture substrate was produced by coating the surface of the cell culture substrate produced in the same manner as in Example 1 (on the side on which the layer containing the hydrophilic polymer is formed) with a block copolymer (temperature-responsive polymer) having a water-insoluble block segment and a temperature-responsive block segment. Specifically, 0.40 g (0.1 mmol) of 4-cyano-4-[(dodecylsulfanylthiocarbonyl)sulfanyl]pentanoic acid, 7.11 g (50 mmol) of n-butyl methacrylate, and 33 mg (0.2 mmol) of azobis(isobutyronitrile) were added to a test tube and dissolved in 50 mL of 1,4-dioxane. After deaeration through nitrogen bubbling for 30 minutes, the deaerated mixture was reacted at 70°C for 24 hours. After the completion of the reaction, the reaction solvent was distilled off under reduced pressure with a rotary evaporator to concentrate the reaction solution. A concentrate was poured into 250 mL of methanol, and a precipitated yellow oily substance was collected and dried under reduced pressure to obtain an n-butyl methacrylate polymer.

0.9 g (0.3 mmol) of the above-obtained n-butyl methacrylate polymer, 8.14 g (72 mmol) of N-isopropyl acrylamide, and 5 mg (0.03 mmol) of azobisisobutyronitrile were added to a test tube and dissolved in 15 mL of 1,4-dioxane. After deaeration through nitrogen bubbling for 30 minutes, the deaerated mixture was reacted at 65°C for 17 hours. After the completion of the reaction, the reaction solvent was diluted with acetone and poured into 500 mL of hexane, and a precipitated solid was collected and dried under reduced pressure. In addition, the resulting solid was dissolved in acetone again and poured into 500 mL of pure water, and a precipitated solid was collected and dried under reduced pressure to obtain a block copolymer of N-isopropyl acrylamide and n-butyl methacrylate. The synthesized block copolymer was dissolved in ethanol at 0.5 wt% and used to spin-coat the surface of the cell culture substrate (on the side on which the layer containing the hydrophilic polymer is formed) at 2,000 rpm.

The structural unit proportion of the block copolymer was 4 wt% for n-butyl methacrylate and 96 wt% for N-isopropyl acrylamide, and the number average molecular weight (Mn) of the block copolymer was 77,000. The layer thickness of the block copolymer (temperature-responsive polymer) layer was determined from a calibration curve in a case where the polystyrene dish was directly coated with the block copolymer (temperature-responsive polymer). As a result, the layer thickness of the block copolymer (temperature-responsive polymer) layer was 25 nm.

The structure of the produced cell culture substrate and evaluation results are shown in Table 2. The produced cell culture substrate had a layer thickness of a hydrophilic polymer layer of 600 nm, an area of a region (A) of 0.03 mm², an unevenness height at a boundary between a region (A) and a region (B) of 4 nm.

Culture of the human iPS cells on this cell culture substrate was evaluated, and the formation of cell aggregates was confirmed two days after the start of culture. In visual assessment, the sizes of the formed cell aggregates were uniform. The height-to-diameter ratio of the cell aggregates was estimated to be 0.5. The cell aggregates were peeled off by cooling the cell culture substrate. In addition, the viability of cells contained in the cell aggregates was 88%, which was a high cell viability. In addition, air bubbles had not adhered to the cell culture substrate during addition of a medium.

### [Example 11]

A cell culture substrate was produced by coating the surface of the cell culture substrate produced in the same manner as in Example 1 (on the side on which the layer containing the hydrophilic polymer is formed) with a random copolymer consisting of a water-insoluble monomer unit and a temperature-responsive monomer unit. Specifically, 0.40 g (0.1 mmol) of 4-cyano-4-[(dodecylsulfanylthiocarbonyl)sulfanyl]pentanoic acid, 7.11 g (50 mmol) of n-butyl methacrylate, 33 mg (0.2 mmol) of azobis(isobutyronitrile), 5.65 g (50 mmol) of N-isopropyl acrylamide, and 5 mg (0.03 mmol) of azobisisobutyronitrile were added to a test tube and dissolved in 15 mL of 1,4-dioxane. After deaeration through nitrogen bubbling for 30 minutes, the deaerated mixture was reacted at 70°C for 24 hours. After the completion of the reaction, the reaction solvent was diluted with acetone and poured into 500 mL of hexane, and a precipitated solid was collected and dried under reduced pressure. In addition, the resulting solid was dissolved in acetone again and poured into 500 mL of pure water, and a precipitated solid was collected and dried under reduced pressure to obtain a random copolymer of N-isopropyl acrylamide and n-butyl methacrylate. The obtained random copolymer was dissolved in ethanol at 0.5 wt% and used to spin-coat the surface of the cell culture substrate (on the side on which the layer containing the hydrophilic polymer is formed) at 2,000 rpm.

The structural unit proportion of the random copolymer was 54 wt% for n-butyl methacrylate and 46 wt% for N-isopropyl acrylamide, and the number average molecular weight (Mn) of the random copolymer was 158,000. The layer thickness of the random copolymer layer was obtained from a calibration curve in a case where the polystyrene dish was directly coated with the random copolymer. As a result, the layer thickness of the random copolymer layer was 25 nm. Although the random copolymer layer does not correspond to the layer of the temperature-responsive polymer, it is listed in the column of the layer of the temperature-responsive polymer in Table 2 for convenience.

The structure of the produced cell culture substrate and evaluation results are shown in Table 2. The produced cell culture substrate had a layer thickness of a hydrophilic polymer layer of 600 nm, an area of a region (A) of 0.03 mm², an unevenness height at a boundary between a region (A) and a region (B) of 4 nm.

Culture of the human iPS cells on this cell culture substrate was evaluated, and the formation of cell aggregates was confirmed two days after the start of culture. In visual assessment, the sizes of the formed cell aggregates were uniform. The height-to-diameter ratio of the cell aggregates was estimated to be 0.5. The cell aggregates were not peeled off by cooling the cell culture substrate. In addition, the viability of cells contained in the cell aggregates was 85%, which was a high cell viability. In addition, air bubbles had not adhered to the cell culture substrate during addition of a medium.

### [Example 12]

A polyethylene terephthalate (PET) film (Cosmoshine, manufactured by Toyobo Co., Ltd.) with a film thickness of 180 µm was subjected to a plasma treatment (under a gas pressure of 20 Pa with a conduction current of 20 mA for an irradiation time of 30 seconds). A water-ethanol solution containing polyvinyl alcohol (BIOSURFINE (R)-AWP, manufactured by Toyo Gosei Co., Ltd.) having an azide group as a hydrophilic polymer at a solid content concentration of 0.1 wt% was used to spin-coat the plasma-treated surface of the above-described PET film at 2,000 rpm. A quartz mask having a plurality of circular chromium-deposited regions with a diameter of 0.2 mm was placed on the hydrophilic polymer layer, and the quartz mask was UV-irradiated from above with a high pressure mercury lamp for 10 seconds. By washing with a mixed solvent of water/ethanol=20/80 to dissolve and remove non-UV-irradiated hydrophilic polymer, regions (regions (A)) having cell adhesiveness and cell proliferation properties were formed. In addition, regions (B) were formed in the UV-irradiated portion. This PET film was bonded to a well plate (a plate having through-holes) having a hollow bottom surface.

The structure of the produced cell culture substrate and evaluation results are shown in Table 2. The produced cell culture substrate had a layer thickness of a hydrophilic polymer layer of 50 nm, an area of a region (A) of 0.03 mm², an unevenness height at a boundary between a region (A) and a region (B) of 50 nm.

Culture of the human iPS cells on this cell culture substrate was evaluated, and the formation of cell aggregates was confirmed two days after the start of culture. In visual assessment, the sizes of the formed cell aggregates were uniform. The height-to-diameter ratio of the cell aggregates was estimated to be 0.5. In addition, the viability of cells contained in the cell aggregates was 90%, which was a high cell viability. In addition, air bubbles had not adhered to the cell culture substrate during addition of a medium.

### [Reference Example 1]

Cell culture substrates were produced in the same manner as in Example 12 except that pure water, a mixed solvent of water/ethanol=80/20, a mixed solvent of water/ethanol=50/50, and ethanol were used instead of the mixed solvent of water/ethanol=20/80 used in the washing step of Example 12.

Table 3 shows results obtained by comparing evaluation of culturing human iPS cells on these cell culture substrates. It was found that the use of a water-ethanol mixed solvent in the washing step can enhance the cell adhesiveness and cell proliferation properties in the regions (A).

### [Reference Example 2]

Cell culture substrates were produced in the same manner as in Example 12 except that a 1.2 mm-thick PS plate, a 0.1 mm-thick polycarbonate (PC) film (Iupilon, manufactured by Mitsubishi Gas Chemical Company, Inc.), a polyethylene (PE) film, a polypropylene (PP) film, and a Permanox slide chamber (manufactured by Thermo Scientific) were used instead of the PET film which was the substrate in Example 12.

Culture of the human iPS cells on these cell culture substrates were evaluated, and the cultured cells were subjected to phase-contrast observation at a magnification of 40 times. In addition, the cells were observed through fluorescent staining, and the comparison results are shown in Table 4. It was found that the regions (A) have excellent cell proliferation properties when the substrates have a polymer containing an alicyclic hydrocarbon group or an aromatic hydrocarbon group in a structural unit. In addition, it was found that clear phase-contrast images of the cells were obtained when the substrates had a refractive index of 1.4 to 1.6 and a thickness of 0.01 to 0.5 mm. Furthermore, it was found that clear fluorescence images of the cells were obtained due to low autofluorescence of the substrates.

### [Comparative Example 1]

A cell culture substrate was produced in the same manner as in Example 4 except that the solid content concentration of the hydrophilic polymer was changed to 0.003 wt%.

The structure of the produced cell culture substrate and evaluation results are shown in Table 2. The produced cell culture substrate had a layer thickness of a hydrophilic polymer layer of 3 nm, an area of a plasma-treated region of 0.03 mm², an unevenness height at a boundary between a plasma-treated region and a non-plasma-treated region of 25 nm.

Culture of the human iPS cells on this cell culture substrate was evaluated, but the cells adhered to or proliferated on the entire surface of the cell culture substrate, whereby no cell aggregates were formed.

### [Comparative Example 2]

A cell culture substrate was produced in the same manner as in Example 4 except that the solid content concentration of the hydrophilic polymer was changed to 3 wt%.

The structure of the produced cell culture substrate and evaluation results are shown in Table 2. The produced cell culture substrate had a layer thickness of a hydrophilic polymer layer of 3,000 nm, an area of a plasma-treated region of 0.03 mm², an unevenness height at a boundary between a plasma-treated region and a non-plasma-treated region of 38 nm.

Culture of the human iPS cells on this cell culture substrate was evaluated, but cell adhesion and proliferation did not occur, whereby no cell aggregates were formed.

### [Comparative Example 3]

The inner surface of a polystyrene (PS) dish with a diameter of 3.5 cm was plasma-treated (under a gas pressure of 20 Pa with a conduction current of 20 mA for an irradiation time of 30 seconds) with a plasma irradiation device (manufactured by Vacuum Device, trade name of Plasma Ion Bombarder PIB-20). 0.8 mL of an ethanol solution containing polyvinyl alcohol (BIOSURFINE (R)-AWP, manufactured by Toyo Gosei Co., Ltd.) having an azide group as a hydrophilic polymer at a solid content concentration of 0.08 wt% was added dropwise onto the plasma-treated PS dish and dried under reduced pressure at room temperature to form a hydrophilic polymer film. A chromium mask having a plurality of circular black patterns with a diameter of 0.2 mm was placed on the hydrophilic polymer film and subjected to UV irradiation to partially cure the hydrophilic polymer. Subsequently, uncured hydrophilic polymer were removed through washing with pure water several times. The structure of the produced cell culture substrate and evaluation results are shown in Table 2.

Culture of the human iPS cells on this cell culture substrate was evaluated, and formation of cell aggregates was confirmed one day after the start of culture. However, the viability of cells contained in the cell aggregates was 65%, which was a low cell viability.

### [Comparative Example 4]

The inner surface of a polystyrene (PS) dish with a diameter of 3.5 cm was plasma-treated (under a gas pressure of 20 Pa with a conduction current of 20 mA for an irradiation time of 30 seconds) with a plasma irradiation device (manufactured by Vacuum Device, trade name of Plasma Ion Bombarder PIB-20). A plurality of circular holes with a diameter of 0.2 mm were formed in a surface protection film (manufactured by Nitto Denko Corporation, E-MASK) through laser processing, and this surface protection film was pasted on the inner surface of the plasma-treated PS dish.

The structure of the produced cell culture substrate and evaluation results are shown in Table 2. The produced cell culture substrate did not have a hydrophilic polymer layer, the area of a plasma-treated region (region which had not been covered with the surface protection film) was 0.03 mm², and the unevenness height at a boundary between a plasma-treated region (region which had not been covered with the surface protection film) and a region which had been covered with the surface protection film was about 58 µm.

Culture of the human iPS cells on this cell culture substrate was evaluated, and formation of cell aggregates was confirmed one day after the start of culture. However, the viability of cells contained in the cell aggregates was 53%, which was a low cell viability. In addition, air bubbles had adhered to the cell culture substrate during addition of a medium.

### [Comparative Example 5]

A cell culture substrate was produced in the same manner as in Example 1 except that a plasma treatment was not performed. The structure of the produced cell culture substrate and evaluation results are shown in Table 2. Culture of the human iPS cells on this cell culture substrate was evaluated, but cell adhesion and proliferation did not occur, whereby no cell aggregates were formed.

### [Comparative Example 6]

A cell culture substrate was produced in the same manner as in Example 1 except that no hydrophilic polymer were used (no hydrophilic polymer layer was formed). The structure of the produced cell culture substrate and evaluation results are shown in Table 2. Culture of the human iPS cells on this cell culture substrate was evaluated, but the cells adhered to or proliferated on the entire surface of the cell culture substrate, whereby no cell aggregates were formed.

**[Table 2]**

| | Substrate | Layer of hydrophilic polymer | | Layer of temperature-responsive polymer | | Region (A) and region (B) | | | Air bubble adhesion evaluation | Evaluation of cell aggregates | | |
|---|---|---|---|---|---|---|---|---|---|---|---|---|
| | | Type of hydrophilic polymer | Layer thickness (nm) | Type of temperature-responsive polymer | Layer thickness (nm) | Plasma treatment on region (A) | Area of region (A) (mm²) | Unevenness height at boundary (nm) | | Whether or not cell aggregates are formed | Cell viability (%) | Detachment of cells due to cooling |
| Example 1 | PS | Azide group-containing polyvinyl alcohol | 600 | - | - | 20 Pa, 30 seconds | 0.03 | 32 | None | Possible | 89% | None |
| Example 2 | PS | Azide group-containing polyvinyl alcohol | 600 | - | - | 20 Pa, 10 minutes | 0.03 | 85 | None | Possible | 91% | None |
| Example 3 | PS | Azide group-containing polyvinyl alcohol | 600 | - | - | 13 Pa, 20 minutes | 0.03 | 452 | None | Possible | 78% | None |
| Example 4 | PS | Phosphorylcholine group-containing polymer | 10 | - | - | 20 Pa, 30 seconds | 0.03 | 23 | None | Possible | 87% | None |
| Example 5 | PS | Phosphorylcholine group-containing polymer | 50 | - | - | 20Pa, 30 seconds | 0.03 | 21 | None | Possible | 92% | None |
| Example 6 | PS | Azide group-containing polyvinyl alcohol | 1500 | - | - | 20Pa, 30 seconds | 0.03 | 34 | None | Possible | 85% | None |
| Example 7 | PET | Azide group-containing polyvinyl alcohol | 600 | - | - | 20Pa, 30 seconds | 0.03 | 38 | None | Possible | 84% | None |
| Example 8 | PS | Azide group-containing polyvinyl alcohol | 600 | - | - | 20Pa, 30 seconds | 1.76 | 35 | None | Possible | 89% | None |
| Example 9 | PS | Phosphorylcholine group- and UV-reactive group-containing polymer | 50 | - | - | 20Pa, 30 seconds | 0.03 | 33 | None | Possible | 86% | None |
| Example 10 | PS | Azide group-containing polyvinyl alcohol | 600 | Poly [N-isopropyl acrylamide]-[butyl methacrylate] | 25 | 20Pa, 30 seconds | 0.03 | 4 | None | Possible | 88% | Done |
| Example 11 | PS | Azide | 600 | Poly(N-isopropyl | 25 | 20Pa, 30 | 0.03 | 4 | None | Possible | 85% | None |

| | | | | | | | | | | | | |
|---|---|---|---|---|---|---|---|---|---|---|---|---|
| | | group-containing polyvinyl alcohol | | acrylamide/butyl methacrylate) | | seconds | | | | | | |
| Comparative Example 1 | PS | Phosphorylcholine group-containing polymer | 3 | - | - | 20Pa, 30 seconds | 0.03 | 25 | None | Impossible | - | - |
| Comparative Example 2 | PS | Phosphorylcholine group-containing polymer | 3000 | - | - | 20Pa, 30 seconds | 0.03 | 38 | None | Impossible | - | - |
| Comparative Example 3 | PS | Azide group-containing polyvinyl alcohol | 800 | - | - | 20Pa, 30 seconds | 0.03 | 798 | None | Possible | 65% | None |
| Comparative Example 4 | PS | - | - | - | - | 20Pa, 30 seconds | 0.03 | about 58 µm | Adhered | Possible | 53% | None |
| Comparative Example 5 | PS | Azide group-containing polyvinyl alcohol | 600 | - | - | - | - | - | None | Impossible | - | - |
| Comparative Example 6 | PS | - | - | - | - | 20Pa, 30 seconds | - | - | None | Impossible | - | - |

**[Table 3]**

| Washing solvent | Region (A) | |
|---|---|---|
| | Cell adhesiveness | Cell proliferation properties |
| Pure water | × | × |
| Water/ethanol=80/20 | ○ | × |
| Water/ethanol=50/50 | ○ | ○ |
| Water/ethanol=20/80 | ○ | ○ |
| Ethanol | × | × |

**[Table 4]**

| Substrate | | | | | | | Cell adhesiveness | Cell proliferation properties | Phase-contrast microscopic image | Fluorescence microscopic image |
|---|---|---|---|---|---|---|---|---|---|---|
| Substrate | Benzene ring structure | Refractive index | Thickness (mm) | Fluorescence intensities | | | | | | |
| | | | | Excitation wavelength of 350 nm | Excitation wavelength of 488 nm | Excitation wavelength of 647 nm | | | | |
| PS | Yes | 1.59 | 1.2 | 1.00 | 1.00 | 1.00 | ○ | ○ | Unclear | Unclear |
| PET | Yes | 1.65 | 0.18 | 6.05 | 209 | 0.90 | ○ | ○ | Unclear | Clear |
| PC | Yes | 1.58 | 0.1 | 0.36 | 0.00 | 0.00 | ○ | ○ | Clear | Clear |
| PE | None | 1.54 | 0.04 | 0.07 | 0.09 | 0.06 | ○ | × | Clear | Clear |
| PP | None | 1.49 | 0.07 | 0.02 | 0.03 | 0.03 | ○ | × | Clear | Clear |
| Permanox | None | - | 1.2 | 3.02 | 5.45 | 9.80 | ○ | × | Unclear | Unclear |

### Reference Signs List

A Region (A)
B Region (B)
H Unevenness height at boundary between region (A) and region (B)
1 Substrate
2 Layer containing a hydrophilic polymer
10, 11 Cell culture substrate
20 Partition plate

**Sequence Listing**

## Claims

1. A cell culture substrate comprising:
a substrate; and
a layer containing a hydrophilic polymer with a layer thickness of 5 to 2,000 nm covering at least a part of a surface of the substrate,
wherein the hydrophilic polymer contain a phosphorylcholine group or a hydroxyl group,
wherein the cell culture substrate has regions (A) below and regions (B) below,
wherein an unevenness height at a boundary between each of the regions (A) and each of the regions (B) is 1 to 500 nm,
wherein each of the regions (A) is an island-shaped region of 0.001 to 5 mm² in area with cell adhesiveness and cell proliferation properties, and
wherein each of the regions (B) is a region which is adjacent to the region (A) and has no cell adhesiveness or cell proliferation properties

2. The cell culture substrate according to claim 1,
wherein the hydrophilic polymer contain a compound represented by General Formula (1) below, a compound represented by General Formula (2) below, or a compound represented by General Formula (3) below, 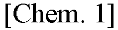 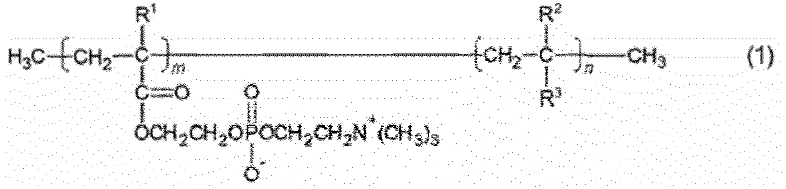 [in General Formula (1), R¹ and R² each independently represent a hydrogen atom or a methyl group, R³ represents a hydrogen atom or an arbitrary organic group, and m and n each independently represent a positive integer], 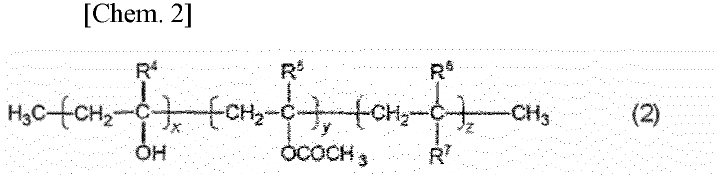 [in General Formula (2), R⁴, R⁵, and R⁶ each independently represent a hydrogen atom or a methyl group, R⁷ represents a hydrogen atom or an arbitrary organic group, and x, y, and z each independently represent a positive integer], and 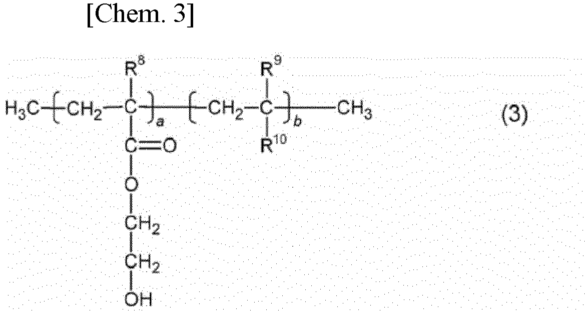 [in General Formula (3), R⁸ and R⁹ each independently represent a hydrogen atom or a methyl group, R¹⁰ represents a hydrogen atom or an arbitrary organic group, and a and b each independently represent a positive integer].

3. The cell culture substrate according to claim 1 or 2,
wherein the hydrophilic polymer contain a monomer unit having a UV-reactive functional group or a residue after a UV reaction.

4. The cell culture substrate according to any one of claims 1 to 3,
wherein a ratio of a peak intensity at 287 eV to a peak intensity at 285 eV in a C1s spectrum of XPS measurement for the regions (A) is greater than a ratio of a peak intensity at 287 eV to a peak intensity at 285 eV in a C1s spectrum of XPS measurement for the regions (B) by 0.05 or more.

5. The cell culture substrate according to any one of claims 1 to 4, further comprising:
a layer containing a temperature-responsive polymer with a layer thickness of 1 to 100 nm on a surface of the layer containing the hydrophilic polymer,
wherein the temperature-responsive polymer is a block copolymer containing a water-insoluble block segment and a temperature-responsive block segment.

6. The cell culture substrate according to claim 5,
wherein a ratio of a weight of the temperature-responsive block segment to a total weight of the water-insoluble block segment and the temperature-responsive block segment is greater than 90 weight%.

7. The cell culture substrate according to any one of claims 1 to 6,
wherein the substrate has a refractive index of 1.4 to 1.6 and a thickness of 0.01 to 0.5 mm.

8. The cell culture substrate according to any one of claims 1 to 7,
wherein fluorescence intensities of the substrate respectively excited at excitation wavelengths of 350 nm, 488 nm, and 647 nm are smaller than fluorescence intensities of a 1.2 mm thick polystyrene plate excited at the same excitation wavelengths.

9. The cell culture substrate according to any one of claims 1 to 8,
wherein each of the regions (A) has an area of 0.005 to 0.2 mm², and
wherein the number of the regions (A) is 200 to 1,000 regions/cm² based on a total area of the regions (A) and the regions (B).

10. The cell culture substrate according to any one of claims 1 to 9,
wherein each of the regions (A) has an area of 0.2 to 2 mm², and
wherein the number of the regions (A) is 3 to 15 regions/cm² based on a total area of the regions (A) and the regions (B).

11. The cell culture substrate according to any one of claims 1 to 10,
wherein minimum distances between the regions (A) are 500 to 10,000 µm.

12. The cell culture substrate according to any one of claims 1 to 11, which is for inducing differentiation from pluripotent stem cells to three germ layer cells.

13. The cell culture substrate according to any one of claims 1 to 12,
wherein the substrate is made of a polycarbonate or a cycloolefin polymer.

14. A method for producing the cell culture substrate according to any one of claims 1 to 13, the method comprising:
Step (1) of coating at least a part of the surface of the substrate with a composition containing a UV-reactive hydrophilic polymer to form a layer containing the hydrophilic polymer;
Step (2) of irradiating the layer containing the hydrophilic polymer with UV light to immobilize the layer containing the hydrophilic polymer on the surface of the substrate; and
Step (3) of subjecting a part of the surface of the immobilized layer containing the hydrophilic polymer to a plasma treatment to form the regions (A) in the plasma-treated portions.

15. A method for producing the cell culture substrate according to any one of claims 1 to 13, the method comprising:
Step (1') of using a substrate made of a polymer containing an alicyclic hydrocarbon group or an aromatic hydrocarbon group in a repeating unit and subjecting the surface of the substrate to a plasma treatment to form the regions (A) in the plasma-treated portions;
Step (2') of coating at least a part of the surface of the substrate with a composition containing UV-reactive hydrophilic polymer to form the layer containing the hydrophilic polymer;
Step (3') of irradiating a part of the layer containing the hydrophilic polymer with UV light to immobilize the part of the layer containing the hydrophilic polymer on the surface of the substrate; and
Step (4') of washing the hydrophilic polymer with a solvent to dissolve hydrophilic polymer that have not been immobilized on the surface and remove them from the surface of the substrate.

16. The method for producing a cell culture substrate according to claim 15,
wherein the solvent used in Step (4') contains water and alcohol.

17. The production method according to any one of claims 14 to 16, further comprising:
Step (4) of bonding a plate having through-holes with a cross-sectional area of 0.05 to 100 cm² in an in-plane direction to the substrate on a surface side coated with the layer containing the hydrophilic polymer of the substrate after Step (3) or (4').

18. The production method according to any one of claims 14 to 17, further comprising:
Step (5) of coating the plasma-treated surface of the layer containing the hydrophilic polymer with a composition containing temperature-responsive polymer to form a layer containing the temperature-responsive polymer after Step (3) or (4').

19. A method for inducing differentiation of pluripotent stem cells, the method comprising:
Step (i) of seeding the cell culture substrate according to any one of claims 1 to 13 with pluripotent stem cells;
Step (ii) of culturing the pluripotent stem cells to form hemispherical cell aggregates with a height-to-diameter ratio of 0.2 to 0.8; and
Step (iii) of inducing differentiation of the cell aggregates to form cell aggregates of three germ layer cells.

20. The method for inducing differentiation of pluripotent stem cells according to claim 19, further comprising:
a step shown in (iv) below,
wherein (iv) is a step of culturing the cell aggregates in a medium containing at least one differentiation-inducing factor selected from the group consisting of a Wnt protein, a bone morphogenetic protein, an insulin-like growth factor, and an activin to express markers possessed by intestinal epithelium cells.

21. A cell culture kit comprising:
the cell culture substrate according to any one of claims 1 to 13; and
block copolymer containing water-insoluble block segment and temperature-responsive block segment or a coating agent containing the block copolymer.
